(19) Europäisches Patentamt
European Patent Office
Office européen des brevets

(11) Publication number: **0 129 409 B1**

(12) **EUROPEAN PATENT SPECIFICATION**

(45) Date of publication of patent specification: **27.10.93**

(51) Int. Cl.5: **C07D 513/04**, A61K 31/425, A61K 31/54, //(C07D513/04, 277:00,235:00),(C07D513/04, 279:00,235:00)

(21) Application number: **84304010.6**

(22) Date of filing: **14.06.84**

The file contains technical information submitted after the application was filed and not included in this specification

(54) **Thiazolo - and thiazinobenzimidazoles.**

(30) Priority: **18.06.83 GB 8316645**
**13.12.83 GB 8333231**

(43) Date of publication of application:
**27.12.84 Bulletin 84/52**

(45) Publication of the grant of the patent:
**27.10.93 Bulletin 93/43**

(84) Designated Contracting States:
**AT BE CH DE FR IT LI LU NL SE**

(56) References cited:

| | |
|---|---|
| **DE-A- 2 155 415** | **DE-C- 242 226** |
| **GB-A- 1 601 335** | **US-A- 3 704 239** |
| **US-A- 3 932 395** | **US-A- 4 214 089** |
| **US-A- 4 293 696** | **US-A- 4 376 769** |

**BULLETIN OF THE CHEMICAL SOCIETY OF JAPAN, vol. 52, no. 10, 1979; R.P. SONI et al. "Synthesis and Absorption Spectra of Some 6-Hydroxythiazolo (3,2-a)benzimidazoles and their Ouaternary Derivatives", pages 3096-3098**

(73) Proprietor: **JOHN WYETH & BROTHER LIMITED**
**Huntercombe Lane South**
**Taplow**
**Maidenhead Berkshire, SL6 0PH(GB)**

(72) Inventor: **Crossley, Roger**
**45 Hazel Drive**
**Woodley Reading Berkshire(GB)**
Inventor: **Meade, Peter Jonathan**
**132 Cookham Road**
**Maidenhead Berkshire(GB)**

(74) Representative: **Wileman, David Francis et al**
**c/o John Wyeth and Brother Limited**
**Huntercombe Lane South**
**Taplow Maidenhead Berkshire SL6 OPH (GB)**

**EP 0 129 409 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS**, vol. 76, no. 25, 19 June 1972, Columbus, Ohio, USA; E.G. KNYSH et al. "Imidazoles. LXVII. Synthesis of derivatives of naphth (1',2':4,5)imidazo(2,1-b)thiazole from 2-mercaptonaphth (1,2-d)imidazole"; page 461, abstract no. 153678w; & Khim. Geterotsikl. Soedin, no 1, 1972, pages 25-29

**CHEMICAL ABSTRACTS**, vol. 95, no. 9, 31 August 1981, Columbus, Ohio, USA; page 793, abstract no. 80996d; & JP-A-81-18989 (S.S. PHARMACEUTICAL CO., LTD.) 23-02-1981

**CHEMICAL ABSTRACTS**, vol. 72, no. 9, 2 March 1970, Columbus, Ohio, USA; H. OGURA et al. "Studies on heterocyclic compounds. IV. Mass spectral study on thiazolo (3,2-a)benzimidazoles"; page 449, abstract no. 43565s & Journal of Heterocyclic Chemistry, vol. 6, no. 6, 1969, pages 797-802

**CHEMICAL ABSTRACTS**, vol. 71, no. 3, 21 July 1969, Columbus, Ohio, USA; P.M. KOCHERIGIN et al. "Synthesis of condensed imidazole system derivatives from 2-haloimidazoles and 8-haloxanthines"; page 329, abstract no. 13065r & Khim. Geterotsikl. Soedin. no. 1, 1969, pages 17

**CHEMICAL ABSTRACTS**, vol. 73, no. 21, 23 November 1970, Columbus, Ohio, USA; A.N. KRASOVSKII et al. "Imididazoles. L. Synthesis of thiazolo(3,2-a)benzimidazole"; page 368, abstract no. 109740z & Khim. Geterotsikl. Soedin. no. 6, 1970, pages 82

**PATENT ABSTRACTS OF JAPAN**, vol. 6, no. 73, 8 May 1982, page (C-101)(951); & JP-A-57-9787 (ESUESU SEIYAKU K.K.) 19-01-1982

## Description

This invention relates to pharmaceutical compositions containing heterocyclic compounds, more particularly benzimidazoles, to novel compounds contained in these compositions and their preparation.

US 4214089 describes thiazolo[3,2-a]benzimidazoles having a phenyl or halophenyl group in the 3-position which are antineoplastic agents.

JP-A-57-9787 describes thiazinobenzimidazoles having a substituted methyl group in position 2 which have vasodilating activity.

6-Hydroxythiazolo[3,2-a]benzimidazoles are described in Bull. Chem. Soc. Japan., 52, 3096-3098 (1979) but no pharmaceutical activity is ascribed to the compounds.

US 3704239 discloses 3-hydroxythiazolo[3,2-a]benzimidazoles having a phenyl or halophenyl group in position 3 which have antitubercular and CNS depressant activity.

US Patent No 4376769 discloses 2,3,5,6-tetrahydro imidazothiazoles and 2,3,6,7-tetrahydrothiazines possessing anti-inflammatory activity. Intermediate 1-hydroxyalkyl-5- substituted imidazolidine -2-thiones are also disclosed as antisecretory agents.

2,3-Dihydrothiazino-benzamidazoles having hypotensive activity are disclosed in Japanese Kokai 8118989 (Chemical Abstracts 95: 80996d). Krasovskii OM in Farm. Zh (Kiev) 1979, (4) 33036 disclosed naphth[1',2':4,5]imidazo[2,1-b]-thiazoles in a study (no data) of compounds with antibacterial and antifungal activities. Thiazolo[3,2-a]benzimidazoles are described in the following Chemical Abstracts references: 72:43565s; 76:52165w; 81:151141v; 73:109740z; 71:22067v; 71:13065r; 76:153678w; 76:153679x and 92:41839y; but no pharmaceutical activity is ascribed to the compounds.

We have now found a series of thiazolo- and thiazino- benzimidazoles which possess pharmaceutical activity, in particular antiulcer activity and/or antisecretory activity and hence are useful as antiulcer agents or for the treatment of gastric hypersecretion. In particular the compounds are useful in the treatment of peptic ulcer disease. The compounds are also useful as intermediates to other compounds in the series.

Accordingly in one aspect this invention provides a pharmaceutical composition comprising a compound of formula

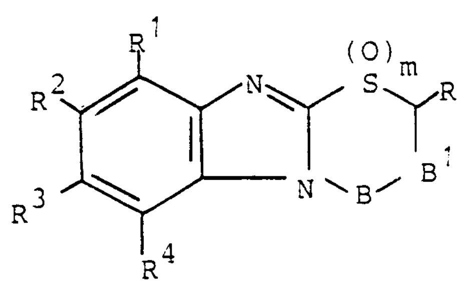

(I)

or a pharmaceutically acceptable salt thereof wherein $-B-B^1-$ represents a chain of formula

$-(CHR^5)n-CHR^6-$ (Ia)

or

$-CR^5=CR^6-$ (Ib)

or

$-(CHR^5)_n-CR^6=$ (Ic)

R represents a mono- or bi-cyclic aryl or heteroaryl radical each optionally substituted by one or more substituents selected from the following list: lower alkyl, lower alkoxy, halogen, alkanoyloxy of 2 to 7 carbon atoms, lower alkoxycarbonyl, halolower alkyl, hydroxy, cyano, amino, mono- or diloweralkyl amino, lower alkanoylamino, carboxy, carboxyloweralkyl, hydroxylower alkyl, carbamoyl, carbamoyloxy, lower alkyl- or aryl-carbonyl, (loweralkoxy)lower alkoxy, or phenyl, or a phenyl group itself optionally substituted by a substituent as hereinbefore defined excepting phenyl; $R^1$, $R^2$, $R^3$ and $R^4$ independently represent hydrogen, or one of the substituents listed above in connection with the group R, $R^2$ and $R^3$ together with the carbon atoms to which they are attached may also complete a fused benzo ring, $R^5$ and $R^6$ independently

represent hydrogen or lower alkyl;n and m independently represent 0 or 1, providing that when -B-$B^1$- has formula I(a), n is 1 and m is O then R is heteroaryl, heteroaryl means a monovalant aromatic heterocyclic group in which the ring heteroatom or atoms is/are selected from O, N and S, the term 'lower' means a group containing 1 to 6 carbon atoms; and a pharmaceutically acceptable carrier.

In a second aspect this invention provides novel compounds of formula I as shown hereinabove or salts thereof, wherein R, $R^1$, $R^2$, $R^3$, $R^4$, m and -B-$B^1$-have the meanings given above with the provisos:

(i) when -B-$B^1$ is a chain of formula Ia, n is 1 and m is O then R is optionally substituted heteroaryl,

or (ii) when -B-$B^1$ is a chain of formula Ic, n is O, $R^6$ is hydrogen or lower alkyl and $R^1$, $R^2$ and $R^4$ are hydrogen, $R^3$ is hydrogen or hydroxy and R is phenyl, p-chlorophenyl, p-bromophenyl, p-tolyl, p-methoxyphenyl, p-phenylphenyl, 1-naphthyl or 2-thienyl, then m is 1.

The term "lower" as used herein to qualify a group means such a group contains 1 to 6 carbon atoms.

Examples of any one of $R^{1-4}$ when substituents are methyl, ethyl,propyl, butyl, isopropyl, methoxy, ethoxy, propoxy, isopropoxy, butoxy, chlorine, bromine, fluorine, acetoxy, propionyloxy, butryloxy, methoxycarbonyl, ethoxycarbonyl, propoxycarbonyl, trifluoromethyl, hydroxy, cyano, amino, methylamino, dimethylamino, ethylamino, acetylamino, carboxy, carboxymethyl, hydroxymethyl, hydroxyethyl, carbamoyl, carbamoyloxy, acetyl,benzoyl or phenyl.

The group R is exemplified by (1) aryl radicals such as phenyl or naphthyl which can be substituted by one or more groups as listed above for any one of $R^{1-4}$, and (2) heteroaryl radicals having one or more heteroatoms selected from oxygen, nitrogen and sulphur, such as pyridyl (e.g. pyrid-2-yl, pyrid-3-yl), thienyl (e.g. thien-2-yl) furyl (e.g. fur-2-yl), thiazolyl e.g. thiazol-2-yl), or bicyclic groups such as quinolyl, isoquinolyl or indolyl,which groups can be substituted by one or more groups as listed above for any one of $R^{1-4}$. Examples of substitutents for R also include (lower alkoxy)- lower alkoxy (e.g. methoxymethoxy, methoxy- and ethoxyethoxy,) phenyl,halophenyl, loweralkylphenyl and loweralkoxyphenyl.

When $R^2$ and $R^3$ form a benzo fused ring the compound of formula I has the general formula

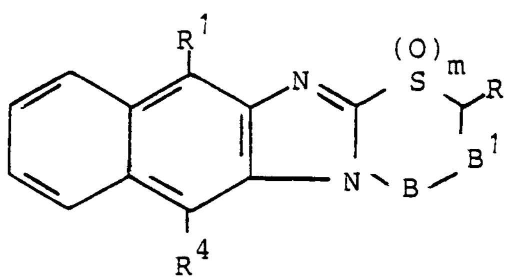

Examples of $R^5$ and $R^6$ when lower alkyl are methyl, ethyl, propyl.

In the compounds of formula I preferred values for -B-$B^1$- are formula Ia or Ic, especially where n is O. Preferably R represents a phenyl or pyridyl group, e.g. pyrid-2- or 3-yl which may be substituted by substituents as hereinbefore described especially lower alkyl, lower alkoxy, and halogen, phenyl, halophenyl, lower alkylphenyl or lower alkoxyphenyl. Preferably m is 1. Preferably either or both $R^2$ and $R^3$ represent substituents selected from lower alkyl (e.g. methyl or ethyl) lower alkoxycarbonyl, (e.g. methoxycarbonyl); halogen (e.g. chlorine or bromine) or $R^2$ and $R^3$ are both hydrogen.

A preferred group of compounds for use in the composition of this invention has the general formula Id

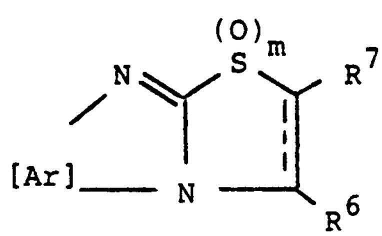

Id

and salts thereof, wherein [Ar] represents

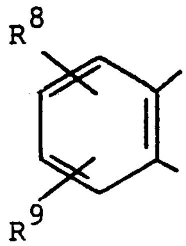

or

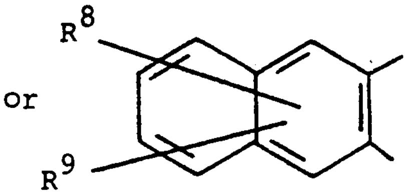

the dotted line represents an optional double bond, $R^7$ is a phenyl or a pyridyl group either of which may be substituted; $R^8$ and $R^9$ independently represent hydrogen or a substituent selected from lower alkyl, lower alkoxy, halogen, cyano, carboxy, loweralkoxycarbonyl, alkanoyloxy of 2 to 7 carbon atoms, carbamoyl, carbamoyloxy, hydroxy, hydroxyalkyl, haloloweralkyl, amino; $R^6$ is as hereinbefore defined and m is 0 or 1.

Preferred compounds of the invention include 2,3-dihydro-2-(2-pyridyl)thiazolo[3,2-a]benzimidazole-1-oxide; 6,7-dichloro-2,3-dihydro-2-(2-methylpyrid-5-yl)thiazolo-[3,2-a]benzimidazole; 2,3-dihydro-6,7-dimethyl-2-(2-pyridyl)-thiazolo[3,2-a]-benzimidazole-1-oxide; 6 or 7-chloro-2,3-dihydro-2-(2-pyridyl)thiazolo[3,2-a]-benzimidazole-1-oxide; 2-(2-(5-ethylpyridyl)thiazolo[3,2-a]-benzimidazole, and 6- or 7-ethoxy-2-(2-pyridyl)-thiazolo-[3,2-a]-benzimidazole.

Examples of acid addition salts are those formed from inorganic and organic acids, in particular pharmaceutically acceptable acid addition salts such as the hydrochloride, hydrobromide, hydroiodide, sulphate, nitrate, phosphate, sulphonate (such as the methanesulphonate and p-toluenesulphonate) acetate, maleate, citrate, fumarate, tartrate, malonate and formate. The salts also include quaternary ammonium salts such as those formed from alkyl or aralkyl halides.

The compounds of formula I possess antiulcer and/or anti-secretory activity as measured by standard test procedures and accordingly are useful for the treatment of ulcers or hypersecretion in mammals.

Anti-ulcer activity was determined by the stress-induced erosion test of Senay and Levine, Proc. Soc. Exp. Biol. Med., 124, 1221-3 (1967). The procedure used was as follows.

Male rats, weighing between 80 and 120 gms. were fasted overnight with water *ad lib*. The rats were then divided into groups of six and dosed orally with the test drug in the form of a solution or with the vehicle alone, 0.5% carboxymethylcellulose, in a volume of 10ml/kg.

After 30 minutes the rats were inserted into aluminium restraining tubes measuring $1^5/_8$ inches in diameter by 5 inches and placed in the cold (4±1 °C) for 3 hours. Immediately after cold exposure the rats were killed with intracranial alcohol and their stomachs excised and opened along the greater curvature. Each stomach was washed gently free of contents with warm tap water and pinned out on a board. The condition of the gastric mucosa was then scored from 0 to 6 on the following scale:

| Ulcers | |
|---|---|
| 0 - 6 | 0 = No ulcers |
| | 1 = Pin point haemorrhagic site |
| | 2) 3) = Several discrete pin point haemorrhagic sites |
| | 4) 5) 6) = Large eroded sites with haemorrhage |

The maximum possible score for each animal was 6 and for the group 36. Decrease in ulcer formation was calculated as a percentage of the control score, i.e.

$$\text{Percentage Inhibition} = \frac{\text{Mean Control group score - Mean Test group score}}{\text{Mean Control group score}} \times 100$$

The statistical significance of the effect is assessed by Student's t-test. Experience has shown that +45% inhibition may be taken as a threshold value below which compounds can be regarded as inactive or not sufficiently active to be considered further.

In the above mentioned test the following representative compounds of formula I were particularly active giving results as shown:

| | Dose (mg/kg) | Inhibition |
|---|---|---|
| 2,3-dihydro-2-(2-pyridyl)-thiazolo[3,2-a]benzimidazole | 100<br>30 | 75%<br>86% |
| 2,3-dihydro-6,7-dimethyl-2-(2-pyridyl)thiazolo[3,2-a]-benzimidazole<br>2,3-dihydro-2-phenylthiazolo-[3,2-a]benzimidazole | 100<br>100 | 82%<br>74% |

Antisecretory activity was demonstrated by the test of H. Shay, D.Sun and H. Gruenstein, Gastroenterology. 1954, 26, 903-13 as exemplified by Beattie et al, J.Med. Chem. 20, 714 (1977). In this test the following representative compounds of formula I were particularly active:

a) 2,3-dihydro-2-(2-pyridyl)thiazolo[3,2-a]benzimidazole and its 1-oxide.

b) 2,3-dihydro-6 or 7-methyl-2-(2-pyridyl)thiazolo[3,2-a]-benzimidazole.

c) 2,3-dihydro-6,7-dimethyl-2-(2-pyridyl)thiazolo[3,2-a]-benzimidazole and its 1-oxide.

d) 2,3-dihydro-6 or 7-methyl-2-(2-pyridyl)thiazolo[3,2-a]-benzimidazole-1-oxide.

e) 2,3-dihydro-6- or 7-chloro-2-(2-pyridyl)thiazolo-[3,2-a]-benzimidazole-1-oxide.

Compounds of formula I were also tested for anti-secretory activity by their ability to inhibit the highly specific proton transporting enzyme $H^+/K^+$ ATPase.

Potential $H^+/K^+$ ATPase inhibitors were evaluated by a technique involving the measurement of aminopyrine accumulation in rabbit isolated gastric glands. Aminopyrine accumulates in acid-secreting cells; therefore, uptake of aminopyrine is increased by secretagogues and an inhibitor of acid secretion will reduce the response to one or more secretagogues depending upon its site of action. Compounds which reduce the response to dibutyryl cyclic adenosine monophosphate (DBcAMP) stimulation are assumed to have an intracellular site of action, and those which reduce the response to both DBcAMP and high potassium ion concentration ($K^+$) are thought to have an' intracellular site of action at the secretory surface of the parietal cell, involving the highly specific proton - transporting enzyme, $H^+/K^+$ ATPase.

The following test procedure is used:

Rabbit gastric glands are isolated from gastric mucosa from the corpus region of the stomach by a method based on one described by Berglindh T., Obrink K.J., Acta Physiol. Scand. 96, 150-159 (1976). Measurement of aminopyrine uptake is carried out using a procedure based on the method described by Berglindh T., Hellander H.F., Obrink K.J. (ibid.97, 401-414, 1976).

Compounds are tested at a concentration of $10^{-4}$ M, initially, and in some cases at lower concentrations, for their ability to inhibit $^{14}$C-aminopyrine uptake in gastric glands, stimulated by DBcAMP and high $K^+$ respectively. Results are expressed as the % inhibition of the maximum response to the secretagogue induced by the test compound. An inhibitor of $H^+/K^+$ ATPase would be expected to reduce the response to both secretagogues.

In the above test the following compounds of formula I were particularly active giving the results shown:

| Compound | % Inhibition to stimulation by: | |
|---|---|---|
| | DBcAMP | $K^+$ |
| 2,3-dihydro-2-(2-pyridyl)-thiazolo[3,2-a]benzimi-dazole-1-oxide | 74% at $10^{-4}$ M<br>73% at $10^{-5}$ M | 91% at $10^{-4}$ M<br>26% at $10^{-5}$ M |
| 6,7-dichloro-2,3-dihydro-2-(6-methylpyrid-3-yl)-thiazolo[3,2-a]benzimidazole | 90% at $10^{-4}$ M | 187% at $10^{-4}$ M |
| 6,7-dichloro-2,3-dihydro-2-( 2-pyridyl)-thiazolo-[3,2-a]-benzimidazole | 50.5% at $10^{-4}$ M | 74% at $10^{-}$M |
| 2,3-dihydro-6,7-dimethyl-2-(2-pyridyl)thiazolo[3,2-a]--benzimidazole-1-oxide | 30% at $10^{-4}$ M | 209% at $10^{-4}$ M |
| 2-(2-(5-ethylpyridyl)-thiazolo[3,2-a]benzimidazole | 56% at $10^{-4}$ M | 142% at $10^{-4}$ M |
| 6- or 7-ethoxy-2-(2-pyridyl)-thiazolo[3,2-a]-benzim-idazole | 68% at $10^{-4}$ M | 162% at $10^{-4}$ M |
| 2-[2-(6-Phenylpyridyl]-thiazolo[3,2-a]benzimidazole | 22% at $10^{-4}$ M | 381% at $10^{-4}$ M |

This invention also provides processes for preparing the novel compounds of formula I. In general the compounds may be prepared by processes which are known or are analogous to known processes - see literature references hereinbefore disclosed.

A first process for preparing compounds of formula I comprises cyclising a compound of formula

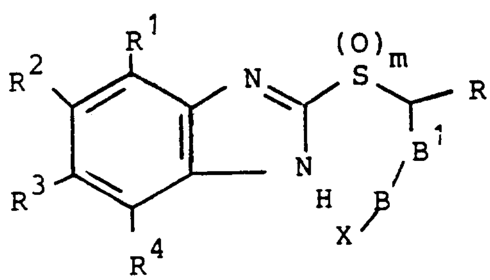

(II)

wherein -B-$B^1$-, n, m, R, $R^1$, $R^2$, $R^3$,$R^4$,$R^5$ and $R^6$ are as defined above, and X is OH or a leaving group such as halogen or an aryl-, alkyl- or aralkyl-sulphonyloxy group that couples B to nitrogen, providing that

(i) when -B-$B^1$- has formula Ia and n is 0 or 1, or formula Ic and n is 1 then X is not OH; and

(ii) when -B-$B^1$- has formula Ib then X is OH.

This cyclisation is conveniently carried out in a suitable solvent if desired under basic conditions (e.g. triethylamine, potassium carbonate) and with heating if required. When X is enolic OH the cyclisation may be carried out in acidic solvent such as acetic anhydride.

Compound of formula II can in general be prepared by reacting an appropriate 2-chlorobenzimidazole with a compound of formula

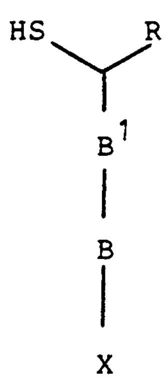

(III)

wherein R, -B-$B^1$- and X are as hereinbefore defined and if desired oxidising the product, e.g. using a peroxyorganic acid such as peroxybenzoic acids.

Compounds of formula II as hereinbefore defined wherein X if OH, $-B-B^1-$ has the formula Ic wherein n us O (enol form of ketone) or formula Ib may be prepared by reacting the appropriate 2-mercaptobenzimidazole with a haloketone or aldehyde of formula IIIa or IIIb.

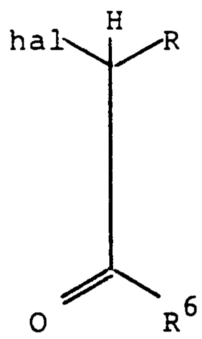

(IIIa)

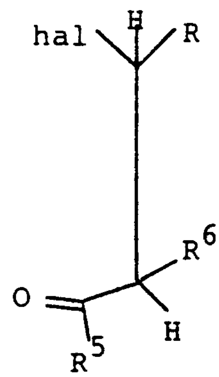

(IIIb)

wherein R, $R^5$ and $R^6$ are as defined above and hal is a halogen. Using this reaction it is possible to go directly to the corresponding compounds of formula I without isolating the intermediates of formula II.

In a preferred process for preparing the compounds of formula I wherein $-B-B^1-$ has formula Ia the compounds of formula II wherein m is 0 are preapred and cyclised without isolation in a single step process by reacting an appropriate 2-mercaptobenzimidazole of formula

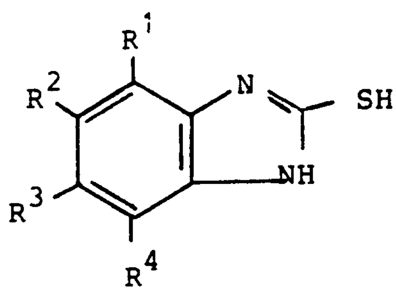

(IV)

wherein $R^1$, $R^2$, $R^3$ and $R^4$ are as defined above with a compound of formula

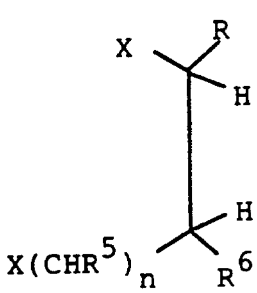

(V)

wherein R, $R^5$ $R^6$ and n are as hereinbefore defined, the X groups being the same or different halogens. This reaction is conveniently carried out by heating in a suitable solvent, e.g. dimethylformamide, if desired in the presence of base. When n is O, and X is bromine then it is possible to isolate from such a reaction a corresponding compound of formula I wherein $-B-B^1-$ is $-(CHR^5)_n-CR^6=$ wherein n is O.

It should be noted that due to tautomerism certain ring substituted 2-mercaptobenzimidazole starting materials are mixtures and hence mixtures of final products are obtained. For example 2-mercapto-5-methylbenzimidazole is tautomeric with 2-mercapto-6-methylbenzimidazole and the final product will be a mixture of compounds where $R^2$ or $R^3$ is methyl.

A further process for preparing the compounds of formula I wherein m is 0 comprises cyclising a compound of formula

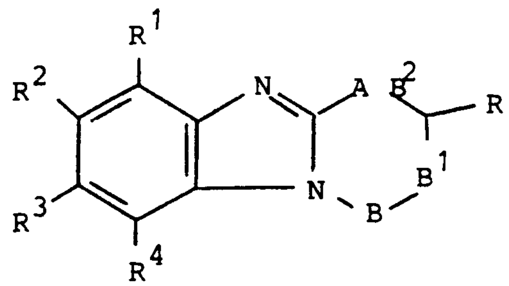

(VI)

wherein -B-$B^1$-, n, R, $R^1$, $R^2$, $R^3$, $R^4$, $R^5$ and $R^6$ are as hereinbefore defined and one of A and $B^2$ is -SH, the other is a leaving group providing that when A is SH then $B^2$ may also represent OH.

When A or $B^2$ is a leaving group the cyclisation is generally carried out by heating if desired in the presence of base, e.g. triethylamine, $K_2CO_3$, NaOH,etc. When $B^2$ is OH t he cyclisation may be carried out in the presence of a strong acid, e.g. HCl or polyphosphoric acid.

Compounds of formula VI wherein A is SH and $B^2$ is OH may be prepared by

a) reacting an appropriate 2-chlorobenzimidazole with a compound of formula

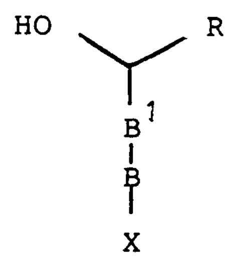

(VII)

wherein -B-$B^1$-, X, R, $R^5$ and $R^6$ are as hereinbefore defined to give a compound of formula VIII

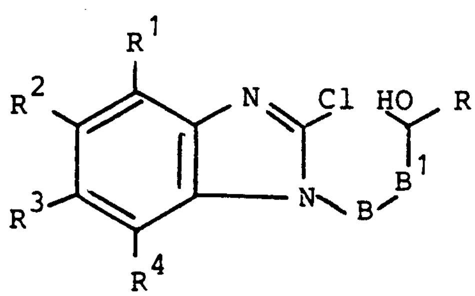

(VIII)

wherein -B-$B^1$-, $R^1$, $R^2$, $R^3$, $R^4$, $R^5$ and $R^6$ are as hereinbefore defined and

b) reacting the compound of formula VIII with thiourea and to give a 2-isothiouronium compound and treating this with an alkali metal hydroxide or ammonium hydroxide under mild conditions, e.g. reacting at room temperature or without heating.

Compounds of formula VIII wherein -B-$B^1$- is $-(CHR^5)_n-CR^6=$ may be reacted in step (b) above under more vigorous conditions, e.g. reflux in a solvent such as a solvent with a boiling point above 50 °C, to give a corresponding compound of formula I directly.

Compounds of formula VI wherein A is SH and $B^2$ is a leaving group and -B-$B^1$- has formula Ia or Ib may be prepared from the corresponding compounds of formula VI wherein $B^2$ is OH by known methods e.g. halogenation, sulphonylation to convert OH to a leaving group.

Compounds of formula VI wherein A is a leaving group such as halogen and $B^2$ is SH may be prepared by building up the molecule from appropriate starting materials wherein the -SH is protected by a thiol protecting group and removing the protecting group as the final step.

Compounds of formula I wherein m is O, -B-$B^1$- has formula Ia and either n is 1 and $R^5$ is hydrogen or n is 0 and $R^6$ is hydrogen may also be prepared by a process which comprises reducing a compound of formula

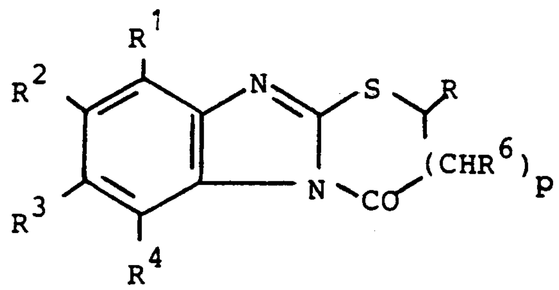

(IX)

wherein R, $R^1$, $R^2$, $R^3$, $R^4$ and $R^6$ are as hereinbefore defined and p is 0 or 1 with the proviso that when p is 1, R is heteroaryl.

This reduction may be carried out using a metal hydride, e.g. lithium aluminium hydride. The compounds of formula IX may be prepared by

cyclising a corresponding compound of formula X

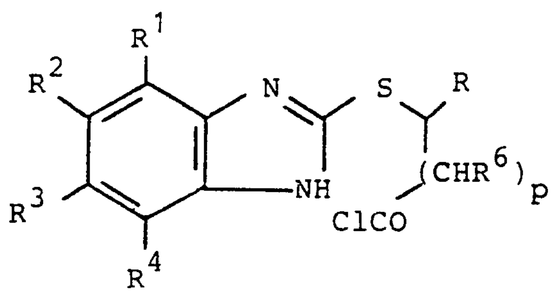

(X)

wherein p, R, $R^1$, $R^2$, $R^3$, $R^4$ and $R^6$ are as defined above.

Compounds of formula X may be prepared by reacting the appropriate 2-mercaptobenzimidazole with a haloacid of formula $RCHBr(CHR^6)_pCOOH$ (R, $R^6$ and p as defined herein) and converting the acid to the acid chloride.

In yet a further process the compounds of formula I wherein m is 0 may be prepared by reacting a compound of formula

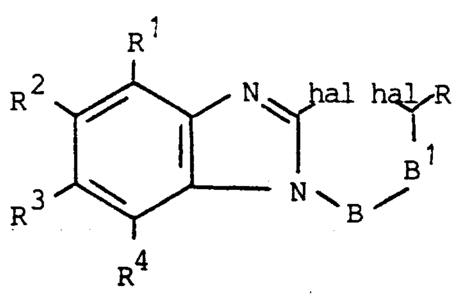

(XI)

wherein -B-$B^1$-, hal, n, R, $R^1$, $R^2$, $R^3$, $R^4$, $R^5$ and $R^6$ are as hereinbefore defined with

(i) an alkali metal sulphide or hydrosulphide,

(ii) ammonium sulphide or polysulphide or

(iii) $H_2S$ in the presence of a tertiary amine.

Yet a further process for preparing compounds of formula I wherein -B-$B^1$- is $-(CHR^5)_{n-}CR^6 =$ comprises cyclizing a compound of formula

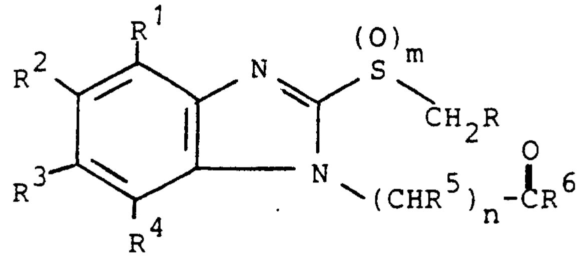

(XIV)

wherein R, $R^1$, $R^2$, $R^3$, $R^4$, $R^5$, $R^6$ and n are as hereinbefore defined. The cyclisation may be conveniently carried out under condensation conditions such as treatment with mixed base/acid systems, e.g. sodium formate/formic acetic anhydride or sodium acetate/acetic anhydride, or by treatment with base followed by subsequent dehydration under acid conditions.

Compounds of formula I wherein m is 0 and 1 may be interconverted. For example when m is 0 the compounds may be oxidised to the corresponding oxides of formula I wherein m is 1 by treatment with suitable oxidising agents e.g. hdyrogen peroxide, sodium periodate, peroxy acids such as peroxybenzoic acids and peroxyalkanoic acids. When m is 1 the compound of formula I may be reduced to the corresponding compound where m is 0 using a reducing agent such as a metal or boron hydride, e.g. $BHCl_2$. Compounds of formula I in which -B-$B^1$- does not contain a double bond may be oxidised to compounds of formula I in which a double bond is present and vice versa by reduction. Accordingly compounds of formula I are intermediates for other compounds of formula I.

The compounds of formula I possess one or more asymmetric centres and hence optical isomers and mixtures thereof are possible. All such isomers and mixtures thereof are included within the scope of this invention. Where any reaction process produces mixtures of such isomers standard resolution techniques may be applied to separate a specific isomer.

In any of the aforementioned reactions compounds of formula I may be isolated in free base form or as acid addition salts as desired. Quaternary ammonium salts may be prepared by reaction with an appropriate halide.

Processes as described hereinabove which prepare novel compounds of formula I are within the scope of this invention.

For the pharmaceutical compositions any suitable carrier known in the art can be used. In such a composition, the carrier may be a solid, liquid or mixture of a solid and a liquid. Solid form compositions include powders, tablets and capsules. A solid carrier can be one or more substances which may also act as flavouring agents, lubricants, solubilisers, suspending agents, binders, or tablet disintegrating agents; it can also be encapsulating material. In powders the carrier is a finely divided solid which is in admixture with the finely divided active ingredient. In tablets the active ingredient is mixed with a carrier having the necessary binding properties in suitable proportions and compacted in the shape and size desired. The powders and tablets preferably contain from 5 to 99, preferably 10-80% of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax and cocoa butter. The term "composition" is intended to include the formulation of an active ingredient with encapsulating material as carrier, to give a capsule in which the active ingredient (with or without other carriers) is surrounded by carriers, which is thus in association with it. Similarly cachets are included.

Sterile liquid form compositions include sterile solutions, suspensions, emulsions, syrups and elixirs. The active ingredient can be dissolved or suspended in a pharmaceutically acceptable carrier, such as sterile water, sterile organic solvent or a mixture of both. The active ingredient can often be dissolved in a suitable organic solvent, for instance aqueous propylene glycol containing from 10 to 75% of the glycol by weight is generally suitable. Other compositions can be made by dispersing the finely-divided active ingredient in aqueous starch or sodium carboxymethyl cellulose solution, or in a suitable oil, for instance arachis oil.

Preferably the pharmaceutical composition is in unit dosage form, the composition is sub-divided in unit doses containing appropriate quantities of the active ingredient; the unit dosage form can be a packaged composition, the package containing specific quantities of compositions, for example packeted powders or vials or ampoules. The unit dosage form can be a capsule, cachet or tablet itself, or it can be the appropriate number of any of these in packaged form. The quantity of active ingredient in a unit dose of composition may be varied or adjusted from 10 to 500 mg or more, e.g. 25 mg to 250 mg, according to the particular need and the activity of the active ingredient. The invention also includes the compounds in the

absence of carrier where the compounds are in unit dosage form.

The anti-ulcer compositions of the invention will be adminsitered orally in either liquid or solid composition form. These compositions may include one or more antacid ingredients, e.g. aluminium hydroxide, magnesium hydroxide or bismuth carbonate, aluminium glycinate, calcium carbonate, magnesium trisilicate, sodium bicarbonate or the alumina gel described in British Specification No. 1,284,394.

In another aspect the invention provides as an anti-ulcer agent a compound of formula I or a pharmaceutically acceptable salt thereof as defined above.

The following examples illustrate the invention:

EXAMPLE 1

2,3-Dihydro-2-(2-pyridyl)thiazolo[3,2-a]benzimidazole

To 2-(1-,2-dibromoethyl)pyridine hydrobromide (10g) dissolved in dimethyl formamide (250ml) was added 2-mercaptobenzimidazole (7g) and the mixture was stirred at 60°C for 36 hours. The solvent was removed under reduced pressure and the residue diluted with 2N HCl (250ml) to give a slight precipitate. This was removed by filtration and the filtrate washed with ether (3 x 150ml), basified ($Na_2CO_3$) and extracted with $CH_2Cl_2$ (4 x 100ml). The extracts were dried ($MgSO_4$) and the solvent removed under reduced pressure. The residue was dissolved in EtOAc to give a slight precipitate which was removed by filtration. The filtrate was concentrated under reduced pressure and the residue dissolved in xylene (500ml) to give a slight precipitate which was removed by filtration. The filtrate was concentrated under reduced pressure and the residue dissolved in methanol (500ml) and treated with decolourising charcoal for 18 hours. The solution was then filtered and the filtrate concentrated under reduced pressure. The residue was purified initially by chromatography on silica using EtOAc as eluent, and finally by h.p.l.c. using 40% EtOAc in $CH_2Cl_2$. The purified material was treated with ethereal hydrogen chloride and recrystallised from propan-2-ol to give the title compound as the di-HCl salt (1.8g) mp 226-229°C decomp. Analysis: Found: C, 51.1; H, 4.1; N, 13.05 $C_{14}H_{11}N_3S.2HCl$ requires: C, 51.5; H, 4.0; N, 12.9%

EXAMPLE 2

2,3-Dihydro-2-(2-pyridyl)thiazolo[3,2-a]benzimidazole-1-oxide

2,3-Dihydro-2-(2-pyridyl)thiazolo[3,2-a]benzimidazole. (2g) was dissolved in ethyl acetate (150ml) and the solution cooled to -50°C. A solution of m-chloroperoxybenzoic acid (1.7g) in ethyl acetate (30ml) was added and the temperature of the mixture allowed to rise to ambient temperature. The mixture was washed with saturated sodium carbonate solution (5ml) and dried ($MgSO_4$). The solvent was concentrated under reduced pressure keeping the temperature below 30°C. The title compound precipitated out as the $\frac{1}{4}$ hydrate, (0.9g). mp 177°C decomp.

Analysis:

Found: C, 61.6; H, 4.2; N, 15.6

$C_{14}H_{11}N_3OS.\frac{1}{4}H_2O$ requires C, 61.35; H, 4.2; N, 15.35%

EXAMPLE 3

2,3-Dihydro-2-phenylthiazolo[3,2-a]benzimidazole

(1,2-Dibromoethyl)benzene (21g) was treated with 2-mercaptobenzimidazole (17g) in dimethylformamide (100ml) and the mixture was heated at 100°C for 36 hours. On cooling, the solid material was removed by filtration and recrystallised from methanol and ethyl acetate to give the title compound as the hydrobromide salt (13.68g). mp 207-210°C.

Analysis:

Found: C, 53.8; H, 4.1; N, 8.5

$C_{15}H_{12}N_2S.HBr$ requires C, 54.1; H, 3.9; N, 8.4%

EXAMPLE 4

2,3-Dihydro-2-phenylthiazolo[3,2-a]benzimidazole-1-oxide

2,3-Dihydro-2-phenylthiazolo[3,2-a]benzimidazole (4.25g) was dissolved in ethyl acetate (250ml) and cooled to -50°C. Solid m-chloroperoxybenzoic acid was added and the mixture allowed to warm to -30°C, at which point saturated sodium carbonate solution (10ml) was added and allowed to freeze.

On reaching ambient temperature the reaction mixture was filtered and dried ($MgSO_4$) and the solvent removed by evaporation to give a white residue. This was recrystallised twice from ethyl acetate to give the title compound as the $\frac{1}{4}$ hydrate (0.75g) mp 162.5-163°C.

Analysis:
Found: C, 65.7; H, 4.7; N, 10.0
$C_{15}H_{12}N_2OS.\frac{1}{4}H_2O$ requires C, 66.0; H, 4.6; N, 10.3%.

EXAMPLE 5

2,3-Dihydro-2-(6-methylpyrid-3-yl)thiazolo[3,2-a]-benzimidazole

3-(1,2-Dibromoethyl)-6-methylpyridine hydrobromide (2.5g) was added to 2-mercaptobenzimidazole (1.25g) in dimethylformamide (50ml). The mixture was stirred at ambient temperature for 4 days and then heated at 100°C for a further 2 days. The solvent was removed under reduced pressure and the residue diluted with 2N HCl, filtered and extracted with ethyl acetate. The aqueous layer was basified with sodium hydroxide and extracted with ethyl acetate (3 x 100ml), dried ($MgSO_4$) and the solvent removed. The residue was purified by chromatography on Fluorisil using 10% cyclohexane in ethyl acetate as eluent. The solvent was removed and the residue dissolved in 25ml ether and ethereal HCl added. The solid obtained was recrystallised from methanol/ethyl acetate to give the title compound as the dihydrochloride, hydrate salt (1.3g) mp greater than 270°C.

Analysis:
Found: C, 49.9; H, 4.45; N, 11.65
$C_{15}H_{13}N_3S.2HCl.H_2O$ requires C, 50.3; H, 4.8; N, 11.7%.

EXAMPLE 6

6,7-Dichloro-2,3-dihydro-2-(6-methylpyrid-3-yl)thiazolo-[3,2-a]benzimidazole

To 5,6-dichloro-2-mercaptobenzimidazole (7.5g) in dimethylformamide (200ml) was added 3-(1,2-dibromoethyl)-6-methylpyridine hydrobromide (7.5g). The mixture was stirred at ambient temperature for 3 days, and then heated at 100°C for 3 days. The solvent was removed by evaporation and the residue diluted with 2N HCl, filtered through keiselghur and extracted with chloroform (3 x 125ml). The aqueous solution was then basified with $Na_2CO_3$ and extracted with dichloromethane (3 x 300ml). The extracts were dried ($MgSO_4$) and the solvent removed under reduced pressure. The residue was washed with ethyl acetate (5 x 2ml), dissolved in hot propan-2-cl and ethereal HCl was added. The solid obtained was recrystallised from methanol/ethyl acetate to give the title compound as the dihydrochloride, $\frac{1}{4}$ hydrate salt (3.1g) mp >325°C.

Analysis:
Found: C, 43.9; H, 3.4; N, 10.00
$C_{15}H_{11}Cl_2N_3S.2HCl.\frac{1}{4}H_2O$ requires C, 43.55; H, 3.3; N, 10.2%

EXAMPLE 7

2,3-Dihydro-6-methyl-2-(2-pyridyl)thiazolo[3,2-a]-benzimidazole and 2,3-Dihydro-7-methyl-2-(2-pyridyl)-thiazolo [3,2-a]benzimidazole

2-(1,2-Dibromoethyl)pyridine hydrobromide (9.5g) was added to 5-methyl-2-mercaptobenzimidazole (5g) in dimethylformamide (100ml) and the mixture stirred at ambient temperature for 3 hours and then heated at 100°C for 24 hours. The solvent was removed and the residue was dissolved in 2N HCl. The resulting solution was filtered through keiselghur and then extracted with ethyl acetate. The aqueous layer was basified ($Na_2CO_3$) and extracted with chloroform. The extracts were dried ($MgSO_4$) and the solvent removed

under reduced pressure. The residue was purified by chromatography on fluorisil with 25% v/v chloroform in ethylacetate and then on silica using 10% v/v hexane in ethylacetate. The solvent was removed and the residue dissolved in hot propan-2-ol and ethereal HCl was added. The product obtained was recrystallised from methanol/ethylacetate to give a mixture of the title compounds as the dihydrochloride hemihydrate salts (1.24g) mp 212-214 °C decomp.

Analysis:
Found: C, 51.4; H, 4.45; N, 12.0
$C_{15}H_{13}N_3S.2HCl.\frac{1}{2}H_2O$ requires C, 51.6; H, 4.6; N, 12.0%.

EXAMPLE 8

6,7-Dichloro-2,3-dihydro-2-(2-pyridyl)thiazolo[3,2-a]-benzimidazole

2-(1,2-Dibromoethyl)pyridine hydrobromide (10.1g) was added to 5,6-dichloro-2-mercaptobenzimidazole (7.1g) in dimethylformamide and the mixture left at ambient temperature for 24 hours, after which it was heated at 100 °C for 3 days. The solvent was removed under reduced pressure and the residue dissolved in 2N HCl and extracted with ethyl acetate. The aqueous layer was basified ($Na_2CO_3$) and extracted with ethyl acetate. The organic extracts were dried ($MgSO_4$) and the solvent removed under reduced pressure. The residue was purified by chromatography on fluorisil using 30% v/v chloroform in ethyl acetate and then on fluorisil using ethyl acetate as eluent. The solvent was removed under reduced pressure and the residue washed with a small volume of ethyl acetate. The residue was then dissolved in hot propan-2-ol and ethereal HCl was added. The solid obtained was filtered and dried to give the title compound as the dihydrochloride salt (1.3g) mp 233-235 °C.

Analysis:
Found: C, 42.5; H, 3.1; N, 10.5
$C_{14}H_9Cl_2N_3S.2HCl$ requires C, 42.6; H, 2.8; N, 10.6%.

EXAMPLE 9

2,3-Dihydro-6,7-dimethyl-2-(2-pyridyl)thiazolo[3,2-a]-benzimidazole

2-(1,2-Dibromoethyl)pyridine hydrobromide (9.7g) was added to 5,6-dimethyl-2-mercaptobenzimidazole (5.3g) in methanol (100ml) and the mixture was heated at reflux for 18 hours, after which time the solvent was removed under reduced pressure. The residue was treated with saturated sodium carbonate solution and extracted into dichloromethane. The extracts were dried ($MgSO_4$) and evaporated and the residue was purified by chromatography on silica using ethyl acetate as eluent. The solvent was removed under reduced pressure and the residue dissolved in hot propan-2-ol and ethereal HCl was added. The product obtained was recrystallised from methanol and ethyl acetate to give the title compound as the dihydrochloride, hemihydrate (0.75g, 7.35%) mp 222 °C decomp.

Analysis:
Found: C, 53.0; H, 4.8; N, 11.3
$C_{16}H_{15}N_3S.2HCl.\frac{1}{2}H_2O$ requires C, 52.9; H, 5.0; N, 11.6%.

EXAMPLE 10

2,3-Dihydro-6-methyl-2-(2-pyridyl)thiazolo[3,2-a]-benzimidazole-1-oxide and 2,3-Dihydro-7-methyl-2-(2-pyridyl)thiazolo[3,2-a]benzimidazole-1-oxide

A mixture of 6- and 7-methyl-2,3-dihydro-2-(2-pyridyl)thiazolo[3,2-a)benzimidazoles (1.57g) was dissolved in ethyl acetate (120ml) and cooled to 0 °C. m-Chloroperoxybenzoic acid (1.09g) was added and the mixture stirred for 1 hour. More m-chloroperoxybenzoic acid (0.1g) was added and the mixture was left a further ½ hour. Saturated sodium carbonate solution was added and the reaction mixture was filtered. The organic layer was dried ($MgSO_4$) and evaporated to low volume. Acetonitrile (0.5ml) was added and the product crystallised. The solid was isolated by filtration and was washed with acetonitrile and with ether to give a mixture of the quarterhydrates of the title compounds (1g) mp 153 °C.

Analysis:
Found: C, 62.5; H, 4.6; N, 14.3
$C_{15}H_{13}N_3OS.\frac{1}{4}H_2O$ requires C, 62.6; H, 4.8; N, 14.6%.

EXAMPLE 11

2,3-Dihydro-6-methoxycarbonyl-2-(2-pyridyl)thiazolo-[3,2-a]benzimidazole and 2,3-Dihydro-7-methoxycarbonyl-2-(2-pyridyl)thiazolo[3,2-a]benzimidazole

Methyl 2-mercaptobenzimidazole-5-carboxylate (13.18g) was suspended in 2-methylpropan-2-ol (180ml) and potassium tert-butoxide (7.08g) was added. The mixture was left to stir 1 hour and then 2-(1,2-dibromoethyl)pyridine hydrobromide (21.82g) was added. Stirring continued for 1 hour at ambient temperature and then 2 hours at reflux.

The mixture was filtered and the solid obtained washed with ether. The filtrate was evaporated to dryness and the residue combined with the solid obtained by filtration and the combined material was dissolved in 2N HCl. The aqueous solution was extracted with ethyl acetate, basified ($Na_2CO_3$) and then extracted with dichloromethane. The organic layer was dried ($MgSO_4$) and evaporated to dryness. The residue was purified by chromatography on fluorisil using chloroform as eluent and then on silica using 1:1 v/v methyl acetate and chloroform. The solvent was removed and the residue recrystallised from methyl acetate/cyclohexane to give a mixture of the title compounds (5.30g) mp 123°C.

Analysis:
Found: C, 61.9; H, 4.4; N, 13.3
$C_{16}H_{13}N_3O_2S$ requires C, 61.7; H, 4.2; N, 13.5%

EXAMPLE 12

2,3-Dihydro-6-methoxycarbonyl-7-methyl-2-(2-pyridyl)-thiazolo[3,2-a]benzimidazole and 2,3-dihydro-7-methoxycarbonyl-6-methyl-2-(2-pyridyl)thiazol[3,2-a]-benzimidazole

2-Mercapto-5-methoxycarbonyl-6-methylbenzimidazole (2.8g) was suspended in 2-methyl-2-propanol (50ml) and potassium tert-butoxide (1.41g) was added. The reaction mixture was stirred under nitrogen for one hour at 30°C. Then 2-(1,2-dibromoethyl)pyridine hydrobromide (4.36g) was added all at once and the reaction mixture stirred at 30°C for 1 hour and then at reflux for 2.5 hours.

The solvent was removed under reduced pressure and the resulting residue treated with 2N HCl (10ml) and water (100ml). Insoluble solid was filtered off. The resulting filtrates were washed with ethyl acetate and basified (2N NaOH) and extracted with dichloromethane. The dichloromethane extract was dried ($MgSO_4$) and evaporated to dryness under reduced pressure. The resulting residue was purified by column chromatography, first, on alumina (grade III) using dichloromethane as the eluent and then on silica using $CH_2Cl_2$/MeOAc (1:1 v/v) as the eluent. The solvents were removed under reduced pressure and the resulting residue treated with methyl acetate (1ml), hexane (1ml) and diethyl ether (1ml). The resulting solid was removed by filtration, washed with a small amount of ether and dried to give a mixture of the title compounds (0.75g) mp 104-106°C.

Analysis:
Found: C, 62.7; H, 4.9; N, 12.6
$C_{17}H_{15}N_3O_2S$ requires C, 62.75; H, 4.65; N, 12.9%

EXAMPLE 13

2,3-Dihydro-6,7-dimethyl-2-(2-pyridyl)thiazolo[3,2-a]-benzimidazole-1-oxide

In a manner analogous to Example 10 2,3-Dihydro-6,7-dimethyl-2-(2-pyridyl)thiazolo-[3,2-a]-benzimidazole (2.63g) was reacted with m-chloroperoxybenzoic acid (1.45g) to give the title compound as the $\frac{1}{4}$ hydrate, mp 177-8°C decomp.

Analysis:
Found: C, 64.0; H, 5.1; N, 13.7
$C_{16}H_{15}N_3OS.\frac{1}{4}H_2O$ requires C, 63.7; H, 5.2; N, 13.9%.

EXAMPLE 14

6-Chloro-2,3-Dihydro-2-(2-pyridyl)thiazolo[3,2-a]-benzimidazole and 7-Chloro-2,3-dihydro-2-(2-pyridyl)-thiazolo[3,2-a]benzimidazole

5-Chloro-2-mercaptobenzimidazole (9.25g) was added to a solution of sodium (1.15g) in ethanol (200ml) and was stirred for 5 minutes. 2-(1,2-Dibromoethyl)pyridine hydrobromide (17.3g) was added and the mixture was heated at reflux for 1.25 hours. Further sodium ethoxide (1 equiv.) was added and the mixture heated at reflux for 3 hours. The reaction mixture was filtered and the solvent evaporated under reduced pressure. The residue was dissolved in chloroform (200ml) and was washed with 2% sodium hydroxide solution (200ml) and brine and then dried ($MgSO_4$). The solvent was removed under reduced pressure and the residue dissolved in ethyl acetate and filtered.
The filtrate was evaporated under reduced pressure and the residue purified by repeated chromatography on silica using ethyl acetate as eluent. The solvent was removed under reduced pressure and the solid obtained was dried under vacuum to give a mixture of the title compounds (1.4g) mp 89.5-93 °C.
Analysis:
Found: C, 58.8; H, 3.6; N, 14.5
$C_{14}H_{10}ClN_3S$ requires C, 58.4; H, 3.5; N, 14.6%.

EXAMPLE 15

7-Ethoxy-2,3-dihydro-2-(2-pyridyl)thiazolo[3,2-a]benzimidazole

5-Ethoxy-2-mercaptobenzimidazole (6.03g) was suspended in 2-methylpropan-2-ol (100ml) at 50 °C. Potassium tert-butoxide (3.48g) was added and the mixture stirred for 1 hour. The temperature was allowed to fall to 35 °C and 2-(1,2-dibromoethyl)pyridine hydrobromide (10.76g) was added. The resulting mixture was stirred for 1 hour at 35 °C and then 2 hours at reflux.

The reaction mixture was filtered and the solid washed with ether. The mother liquors were evaporated under reduced pressure and the residue was combined with the solid obtained by filtration. The combined solids were dissolved in 2N HCl and washed with ethyl acetate (4 x 75ml), basified ($Na_2CO_3$) and extracted with dichloromethane (3 x 100ml). The organic layer was dried ($MgSO_4$) and evaporated under reduced pressure. The residue was purified by chromatography on fluorisil using chloroform as eluent. The solvent was removed under reduced pressure when the residue crystallised to give the title compound (1.2g) mp 164-166 °C.
Analysis:
Found: C, 64.4; H, 5.2; N, 14.3
$C_{16}H_{15}N_3OS$ requires C, 64.6; H, 5.1; N, 14.1%.

EXAMPLE 16

2,3-Dihydro-7-methoxycarbonyl-6-methyl-2-(2-pyridyl)-thiazolo[3,2-a]benzimidazole-1-oxide and 2,3-dihydro-6-methoxycarbonyl-7-methyl-2-(2-pyridyl)thiazolo[3,2-a]-benzimidazole-1-oxide

In a manner analogous to Example 10 a solution of 2,3-dihydro-6-methoxycarbonyl-7-methyl-2-(2-pyridyl) thiazolo[3,2-a]benzimidazole and 2,3-dihydro-7-methoxycarbonyl-6-methyl-2-(2-pyridyl)-thiazolo[3,2-a]benzimidazole (0.65g) in dichloromethane (15ml) was reacted with m-chloroperoxybenzoic acid (0.43g) to give a mixture of the title compounds (117mg) 150-151 °C.
Analysis:
Found: C, 59.7; H, 4.7; N, 12.40
$C_{17}H_{15}N_3O_3S$ requires C, 59.8; H, 4.4; N, 12.3%.

EXAMPLE 17

2,3-Dihydro-6-methoxycarbonyl-2-(2-pyridyl)thiazolo[3,2-a]-benzimidazole-1-oxide and 2,3-dihydro-7-methoxycarbonyl-2-(2-pyridyl)thiazolo[3,2-a]benzimidazole-1-oxide

In a manner analogous to Example 10 a mixture of 2,3-dihydro-6-[and 7]-methoxycarbonyl-2-(2-pyridyl) thiazolo[3,2-a]benzimidazole (prepared according to Example 12, 1.81g) was reacted with m-chloroperoxybenzoic acid (1.16g) to give a mixture of the title compounds (500mg) mp 128-35 ° C.
Analysis:
Found: C, 58.5; H, 4.3; N, 12.6
$C_{16}N_{13}N_3O_3S$ requires C, 58.7; H, 4.0; N, 12.8%.

EXAMPLE 18

6-Chloro-2,3-dihydro-2-(2-pyridyl)thiazolo[3,2-a]-benzimidazole-1-oxide and 7-chloro-2,3-dihydro-2-(2-pyridyl)-thiazolo[3,2-a]benzimidazole-1-oxide

In a manner analogous to Example 10 a mixture of 6- and 7-chloro-2,3-dihydro-2-(2-pyridyl)-thiazolo [3,2-a]benzimidazole (prepared according to Example 13, 2.62g) was reacted with m-chloroperoxybenzoic acid (1.81g) to give a mixture of the title compounds (1.9g) mp 163.5-4.5 ° C.
Analysis:
Found: C, 55.6; H, 3.6; N, 14.0
$C_{14}H_{10}ClN_3OS$ requires C, 55.4; H, 3.3; N, 13.8%.

EXAMPLE 19

Using a procedure analogous to Example 1 involving reaction of compounds of formulae IV and V

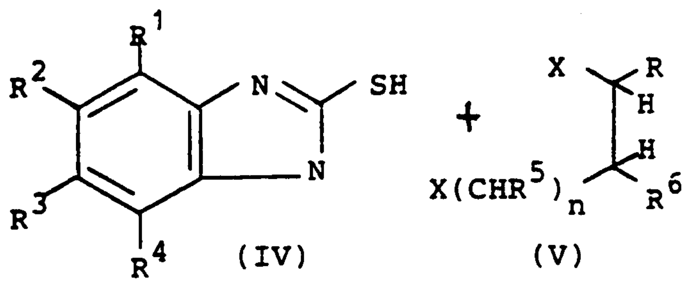

(IV) (V)

wherein X is Cl or Br, the following compounds of formula I wherein m is 0 and -B-B[1]- is $-(CHR^5)_n-CHR^6-$ are prepared

| $R^1$ | $R^2$ | $R^3$ | $R^4$ | R | n | $R^6$ | $R^5$ |
|---|---|---|---|---|---|---|---|
| H | H | H | H | 2-pyridyl | 1 | H | H |
| Me | H | H | Me | 2-pyridyl | 0 | H | - |
| H | $CO_2Me$ | $CO_2Me$ | H | 2-pyridyl | 0 | H | - |
| H | H | H | H | 4-chloro-2-pyridyl | 0 | H | - |
| H | H | H | H | 2-thienyl | 0 | H | - |
| H | H | H | H | 4-pyridyl | 0 | H | - |
| H | H | H | H | 2-pyrimidinyl | 0 | H | - |
| H | H | H | H | 3-chlorophenyl | 0 | H | - |
| H | H | H | H | 4-methylphenyl | 0 | H | - |
| H | H | H | H | 4-methoxyphenyl | 0 | H | - |
| H | H | H | H | 4-methoxycarbonyl-phenyl | 0 | H | - |
| H | H | H | H | 2-acetoxyphenyl | 0 | H | - |

which compounds are all converted to their S-oxides by a method analogous to Example 2.

EXAMPLE 20

2,3-Dihydro-5-methyl-2-(2-pyridyl)thiazolo[3,2-a]-benzimidazole

4-Methyl-2-mercaptobenzimidazole (13.32g) was suspended in 2-methylpropan-2-ol (150ml) and potassium tert-butoxide (9.04g) added. The mixture was stirred for 2 hours and 2-(1,2-dibromoethyl)pyridine hydrobromide (27.68g) added. The mixture was stirred at ambient temperature for 1 hour and then at reflux

for 2 hours. The reaction mixture was filtered and the solid washed with ether. The filtrate was evaporated to dryness under reduced pressure and the residue combined with the first solid obtained. The combined material was dissolved in 2N HCl (100ml). The aqueous solution was extracted with ethyl acetate, basified ($Na_2CO_3$) and extracted with dichloromethane. The organic layer was dried ($MgSO_4$) and the solvent removed under reduced pressure. The residue was purified on Florisil using chloroform as eluent and on silica using ethyl acetate as eluent to give the title compound (1.55g) mp 128-130 °C
Analysis:
Found: C, 67.1; H, 5.0; N, 16.1;
$C_{15}H_{13}N_3S$ requires: C, 67.4; H, 4.9; N, 15.7%.
Florisil is a Registered Trade Mark

EXAMPLE 21

2,3-Dihydro-8-methyl-2-(2-pyridyl)thiazolo [3,2-a]-benzimidazole

Also isolated from the purification step in Example 20 was the title compound (1.5g) m.p. 96 °C.
Analysis:
Found: C, 67.05; H, 4.9; N, 15.7;
$C_{15}H_{13}N_3S$ requires: C, 67.4; H, 4.9; N, 15.7%

EXAMPLE 22

2,3-Dihydro-5-methyl-2-(2-pyridyl)thiazolo[3,2-a]-benzimidazole-1-oxide

In a similar manner to Example 4 2,3-dihydro-5-methyl-2-(2-pyridyl)thiazolo[3,2-a]-benzimidazole (1.07g) was reacted with m-chloroperoxybenzoic acid to give the title compound (0.74g, 70%) mp 148.5 °C.
Analysis:
Found: C, 63.4; H, 4.6; N, 14.7;
$C_{15}H_{13}N_3OS$ requires: C, 63.6; H, 4.6; N, 14.8%.

EXAMPLE 23

2,3-Dihydro-8-methyl-2-(2-pyridyl)thiazolo[3,2-a]-benzimidazole-1-oxide

In a manner analogous to Example 4 2,3-dihydro-8-methyl -2-(2-pyridyl)thiazolo[3,2-a]-benzimidazole (1.04g) was reacted with m-chloroperoxybenzoic acid (0.85g) to give the title compound (0.83g, 80%) mp 182 °C decomp.
Analysis:
Found: 63.2; H, 4.6; N, 15.0;
$C_{15}H_{13}N_3OS$ requires C, 63.6; H, 4.6; N, 14.8%.

EXAMPLE 24

6-Ethoxy-2-(2-pyridyl)thiazolo[3,2-a]-benzimidazole and 7-ethoxy-2-(2-pyridyl)thiazolo[3,2-a]benzimidazole

5-Ethoxy-2-mercaptobenzimidazole (10.43g) was dissolved in 2-methyl-2-propanol (250ml) at 30 °C. Potassium tert-butoxide (5.95g) was added and the mixture was left to stir for 1 hour. 2-(1,2-Dibromoethyl)-pyridine hydrobromide (18.4g) was added and the mixture was stirred at 30 °C for 1 hour and at reflux for 2 hours. The mixture was filtered and the filtrate was evaporated under reduced pressure. The residue was combined with the first solid obtained and was dissolved in 2NHCl. The aqueous solution was extracted with ethyl acetate, basified ($NaCO_3$) and extracted with dichloromethane. The organic layer was dried ($MgSO_4$) and the solvent removed under reduced pressure. The residue was purified by chromatography on Florisil using chloroform as eluent and then on silica using ethyl acetate as eluent. The solvent was removed to give a mixture of the title compounds (0.2g) mp 150-151 °C.
Analysis:
Found: C, 65.3; H, 4.1; N, 14.6;
$C_{16}H_{13}N_3OS$ requires: C, 65.1; H, 4.4; N, 14.2%.

EXAMPLE 25

7-Ethoxy-2,3-dihydro-2-(2-pyridyl)thiazolo [3,2-a]-benzimidazole-1-oxide

In a manner analogous to Example 4 7-ethoxy-2,3-dihydro-2-(2-pyridyl)thiazolo[3,2-a]-benzimidazole (1.65g) was reacted with m-chloroperoxy benzoic acid (1.25) to give the title compound (0.56g, mp 145-147°C.
Analysis:
Found: C, 61.3; H, 4.8; N, 13.7;
$C_{16}H_{15}N_3O_2S$ requires: C, 61.3; H, 4.8; N, 13.4%.

EXAMPLE 26

2,3-Dihydro-2-(2-(5-ethylpyridyl)) thiazolo[3,2-a]-benzimidazole

a)5-Ethyl-2-vinylpyridine (12.0g) (J.Amer.Chem.Soc., 68 1368 (1946)) was dissolved in dichloromethane (40ml) and cooled to ice temperature with stirring. Then bromine (15.8g) dissolved in dichloromethane (30ml) was added dropwise over a period of 5 minutes and the reaction mixture stirred for further 10 minutes at ice temperature.

Anhydrous HBr gas was bubbled through the reaction solution in excess amount and solvent was removed under reduced pressure. The resulting residue was treated with propan-2-ol (10ml) and the resulting yellow solid filtered, washed with ether and dried to yield 2-(1,2-dibromoethyl)-5-ethylpyridine hydrobromide (29.0g) (86%).

b)2-Mercaptobenzimidazole (10.84g) was suspended in 2-methylpropan-2-ol (170ml) and potassium tert-butoxide (8.1g) in 2-methylpropan-2-ol (70ml) added and the reaction mixture stirred under nitrogen for 1 hour at 50°C. The reaction was then cooled to room temperature and 2-(1,2-dibromoethyl)-5-ethylpyridine hydrobromide (27.0g) was added at once and the reaction mixture stirred at room temperature for 2 hours and then refluxed for 2 hours. Further potassium tert-butoxide (8.1g) was added and the reaction mixture refluxed for 2.5 hours.

The solvent was removed under reduced pressure and the resulting residue treated with 2N HCl (200 ml) and water (100 ml) and the insoluble solid filtered off.

The resulting filtrate was washed with ethyl acetate and basified (2N NaOH) and extracted with dichloromethane. The dichloromethane extracts were dried ($MgSO_4$) and evaporated to dryness under reduced pressure.

The resulting residue was purified by column chromatography, first, on alumina (grade III) using dichloromethane as the eluent and then on silica using EtOAc/hexane (85:15 v/v) as the eluent. The solvents were removed under reduced pressure from the fractions having Rf value about 0.29 and the resulting residue treated with methyl acetate (2 ml). The resulting white solid was removed by filtration, washed with a small amount of hexane and dried to give the title compound (7.4g). m.p. 101-102°C.
Analysis:
Found: C, 68.6; H, 5.4; N, 14.7.
$C_{16}H_{15}N_3S$ requires C, 68.3; H, 5.4; N, 14.9%.

EXAMPLE 27

2-(2-(5-Ethylpyridyl))thiazolo[3,2-a]benzimidazole

Solvent was removed from the fractions from the previous example having an Rf value about 0.44 and the resulting residue treated with methyl acetate (2ml). The resulting solid was removed by filtration, washed with small amount of hexane and dried to give title compound (0.4g) m.p. 174-175°C.
Analysis:
Found: C, 68.7; H, 4.9; N, 14.7.
$C_{16}H_{13}N_3S$ requires C, 68.8; H, 4.7; N, 15.0%.

EXAMPLE 28

2,3-Dihydro-2-(2-(5-ethylpyridyl)) thiazolo [3,2-a]-benzimidizole-1-oxide

In a manner analogous to Example 4 2,3-dihydro-2-(2-(5-ethylpyridyl)) thiazolo[3,2-a] benzimidazole was reacted with m-chloroperoxybenzoic acid (1.2g) to give the title compound (0.91g) m.p. 154-155 °C.
Analysis:
Found: C, 64.5; H,5.3; N, 13.7;
$C_{16}H_{15}N_3OS$ requires C, 64.6; H, 5.1; N, 14.1%.

EXAMPLE 29

6-Cyano-2,3-dihydro-2-(2-pyridyl)thiazolo[3,2-a]benzimizazole and 7-Cyano-2,3-dihydro-2-(2-pyridyl)thiazolo-[3,2-a]benzimidazole

In a manner analogous to Example 12, 5-cyano-2-mercaptobenzimidazole (10.46g) was reacted with 2-(1,2-dibromoethyl)pyridine hydrobromide (20.76g) in the presence of potassium tert-butoxide (15.46g) to give the title compound (0.24g), m.p. 158-160 °C.
Analysis:
Found: C, 64.4; H, 3.6; N, 19.85; $C_{15}H_{10}N_4S$
requires: C, 64.7; H, 3.6; N, 20.1%.

EXAMPLE 30

2,3-Dihydro-2-[2-(4-methoxypyridyl)]thiazolo[3,2-a]-benzimidazole

(a) 2-Bromoacetyl-4-methoxypyridine (11.5g, 0.05) and 2-chlorobenzimidazole (7.5g, 0.05 mole) were dissolved in dimethylformamide (75ml) and cooled to 2 °C. $K_2CO_3$ (12g, 0.08 mole) was added and the temperature rose to 9 °C as the suspension was stirred for ½ hour. The mixture was added to $H_2O$ (200 ml) giving a solid which was removed by filtration. The mother liquors were extracted with ethyl acetate and the solid was dissolved in the extracts. The organic solution was washed (brine) and dried ($MgSO_4$) and purified by passage down a silica column and evaporated to dryness to give a sticky solid. Trituration with propan-2-ol/di-isopropyl ether gave 2-chloro-1-[2-(2-[4- methoxypyridyl])-2-oxoethyl]-benzimidazole (7.2g).
(b) A mixture of the product of step (a) (7.2g) and thiourea (2.4g) in ethanol (50ml) was stirred at ambient temperature for ¼ hour and heated at 60 °C for 2 hours. The solution was filtered and evaporated and the residue was dissolved in water and treated to excess with $NH_4OH$ to give a solid which was purified by chromatography on silica with ethyl acetate to give 2-mercapto-1-(2-(2-(4-methoxypyridyl))-2-oxoethyl)-benzimidazole (2g.).
(c) The product of step (b) was dissolved in 0.1N NaOH (70 ml) and ethanol (30 ml) and the solution was treated with $NaBH_4$ (0.3g.). After stirring at ambient temperature for 1 hour, the resulting solution was treated with charcoal, filtered and evaporated to low volume. The residue was acidified to pH5 with acetic acid and the aqueous solution decanted. The residue was crystallised from propan-2-ol to give 1-[2-hydroxy-2-(2-(4-methoxypyridyl))ethyl]-2-mercaptobenzimidazole (1.1g.).
(d) The product of step (c) (0.5g.) was dissolved in polyphosphoric acid (1.7g) and was heated at 120 °C for 1½ hour with stirring. Further 1-[2-hydroxy-2-(2-(4-methoxypyridyl)ethyl]-2-mercaptobenzimidazole (0.5g.) was added and the heating continued 1½ hours. The solution was basified with $Na_2CO_3$ solution to give a solid which was extracted with ethyl acetate. The extracts were dried ($MgSO_4$) and evaporated and the residue triturated with di-isopropyl ether to give the title compound (0.85g.) mp 107-8 °C.
Analysis:
Found: C, 63.5; H, 4.8; N, 14.5;
$C_{15}H_{13}N_3OS$ requires C, 63.6; H, 4.6; N, 14.8%.

EXAMPLE 31

2-(2-Pyridyl)thiazolo[3,2-a]benzimidazole

A solution of 2-mercapto-1-[2-oxo-2-(2-pyridyl)ethyl]benzimidazole (1g.) in polyphosphoric acid was heated at 140°C for 1 hour. The solution was neutralised with $Na_2CO_3$ solution and extracted into EtOAc (700 ml). The extracts were dried ($MgSO_4$) and evaporated to give a solid which was recrystallised from EtOAc/EtOH to give the title compound (0.35g.) mp 222-4°C.
Analysis:
Found: C, 67.3; H, 3.5; N, 16.4.
$C_{14}H_9N_3S$ requires C, 66.9; H, 3.6; N, 16.7%.

EXAMPLE 32

2,3-Dihydro-2-[2-(6-methylpyridyl]thiazolo [3,2-a]-benzimidazole

In a manner analogous to Example 30 steps a) →d) 2-bromoacetyl-6-methylpyridine (11.4g) was reacted with 2-chlorobenzimidazole (8.2g) to give 1-[2-(6-methylpyrid-2-yl)-2-oxoethyl]-2-chlorobenzimidazole (8.4g). This was reacted with thiourea, then $NH_4OH$ to give 1-[2-(6-methylpyrid-2-yl)-2-oxoethyl]-2-mercaptobenzimidazole (6.9g.); which compound was reduced with $NaBH_4$ to give 1-[2-hydroxy-2-(6-methylpyrid-2-yl)-ethyl]-2-mercaptobenzimidazole (5.0g.). Treatment of this with polyphosphoric acid gave the title compound (2.1g.) m.p. 79-81° C.
Analysis:
Found: C, 67.0; H, 4.8; N, 15.6% . $C_{15}H_{13}N_3S$
requires: C, 67.4; H, 4.9; N, 15.7%.

EXAMPLE 33

2,3-Dihydro-2-[2-(6-methylpyridyl)]thiazolo[3,2-a]-benzamidazole-1-oxide

In a manner analogous to Example 4 2,3-dihydro-2-[2-(6-methylpyridyl)thiazolo[3,2-a]benzimidazole (1.0g.) was reacted with m-chloroperoxybenzoic acid (0.71g.) to give the title compound as the quarterhydrate, 0.9g, m.p. 175-177°C.
Analysis:
Found: C, 62.2; H, 4.4; 14.4%.
$C_{15}H_{13}N_3OS.\ \frac{1}{4}\ H_2O$
Requires: C, 62.6; H, 4.7; N, 14.6%.

EXAMPLE 34

2-[2-(6-Methylpyridyl)]thiazolo[3,2-a]benzimidazole

In a manner analogous to Example 31 1-[2-(6-Methylpyrid-2-yl)-2-oxoethyl-2-mercaptobenzimidazole (1.7g) (prepared according to Example 32) was reacted with polyphosphoric acid (20g) to give the title compound as the $\frac{1}{4}$ hydrate, (0.8g,), m.p. 235-7°C
Analysis:
Found: C, 67.15; H, 4.1; N, 15.4%
$C_{15}H_{11}N_3S.\ \frac{1}{4}H_2O$ requires: C, 66.8; H, 4.3;N, 15.6%.

EXAMPLE 35

2,3-Dihydro-2-(2-pyridyl)naphth[2',3':4,5]imidazo-[2,1-b]thiazole

In a manner analogous to Example 30 steps a) →d) 2-chloro-1H-naphtho[2,3-d]imidazole (7.5g) was reacted with 2-bromoacetylpyridine (8.7g) to give 2-chloro-1-(2-oxo-2-(2-pyridyl)ethyl)naphtho[2,3-d]-imidazole (9g). This was reacted with thiourea (5.5g) then $NH_4OH$ solution (100 ml) to give 2-mercapto-1-(2-oxo-2-(2-pyridyl)ethyl)naphtho[2,3-d]imidazo;which compound was reduced with $NaBH_4$ to give 2-mercapto-1-(2-hydroxy-2-(2-pyridyl)ethyl)naphtho[2,3-a]imidazole (1.3g). Treatment of this with polyphosphoric acid

(23g) gave the title compound (0.75g.) mp 197.5-198.5 ° C.
Analysis:
Found: C, 71.2; H, 4.25; N, 13.5; $C_{18}H_{13}N_3S$ requires: C, 71.3; H, 4.3; N, 13.85%).

EXAMPLE 36

2,3-Dihydro-2-[2-(6-phenylpyridyl)]thiazolo[3,2-a]benzimidazole

In a similar manner to Example 30 steps a)→d) 2-bromoacetyl-6-phenyl pyridine (14.1g,) is converted to the title compound via the following intermediates: 1-[2-(6-phenylpyrid-2-yl)-2-oxoethyl]-2-chlorobenzimidazole, 1-[2-(6-phenylpyrid-2-yl)-2-oxoethyl]-2-mercaptobenzimidazole and 1-[2-hydroxy-2-(6-phenylpyrid-2-yl)ethyl]-2-mercaptobenzimidazole, m.p. of the title compound = 145-147 ° C.
Analysis:
Found: C, 73.25; H, 4.9; N, 13.1%
$C_{20}H_{15}N_3S$ requires C,72.9; H, 4.6; N, 12.75%.

EXAMPLE 37

2-[2-(6-Phenylpyridyl)]thiazolo[3,2-a]benzimidazole

In a manner analogous to Example 31 1-[2-(6-Phenylpyrid-2-yl)-2-oxoethyl]-2-mercaptobenzimidazole (2.0g) (prepared as intermediate in Example 36) is reacted with polyphosphoric acid to give the title compound (1.7g,) m.p. 267-268 ° C.
Analysis:
Found: C, 73.3; H, 4.0; N, 12.5%
$C_{20}H_{13}N_3S$ requires: C, 73.4; H, 4.0: N,12.8%.

EXAMPLE 38

2-(2-Pyridyl)naphth[2',3':4,5]imidazo[2,1-b]thiazole

In a manner analogous to Example 31 1-(2-(2-pyridyl)-2-oxoethyl)-2-mercaptonaphtho[2,3-d]imidazole (1g) prepared as an intermediate in Example 35 was reacted with polyphosphoric acid to give the title compound (290 mg) mp 261.5 ° C. decomp.
Analysis:
Found: C, 71.6; H, 3.6; N, 13.7;
$C_{18}H_{11}N_3S$ requires C, 71.7; H, 3.7; N, 13.9%.

EXAMPLE 39

(R*R*)-2,3-dihydro-2-(4-methoxypyrid-2-yl)thiazolo[3,2-a]benzimidazole-1-oxide and its (R*,S*) isomer

A solution of 2,3-dihydro-2-[2-(4-methoxypyridyl)]thiazolo[3,2-a]benzimidazole (0.6g) in $CH_2Cl_2$ (30ml) was cooled to 0 ° . Purified m-chloroperoxybenzoic acid (0.4g) was added and the mixture was stirred 1½hr. Further m-chloroperoxybenzoic acid (0.05g) was added and the mixture was stirred 1 hour and washed ($Na_2CO_3$ solution and brine), dried ($MgSO_4$) and evaporated. The residue was purified by chromatography on silica with EtOH/EtOAc as eluent followed by recrystallisation from acetonitrile to give as a first product the R*R* isomer of the title compound (105mg) mp 193-5 ° C.
Analysis:
Found: C, 60.5; H, 4.5; N, 14.1;
$C_{15}H_{13}N_3O_2S$ requires: C, 60.2; N, 4.4; H, 14.0%.

A further component of lower $R_f$ was isolated and recrystallised from acetonitrile to give the corresponding R*S* isomer of the title compound (90% pure) (115 mg) mp 172-7 ° C.

EXAMPLE 40

2,3-Dihydro-2-(2-pyridyl)thiazino[3,2-a]benzimidazole

a) 3-(2-Benzimidazolylthio)-3-(2-pyridyl)propionic acid (3.7g) was suspended in dichloromethane (100 ml) and was cooled to 0 °C. N,N'-Dicyclohexylcarbodiimide (2.55g) was added and the mixture was left to stir until t.l.c. showed no starting material. The solvent was removed under reduced pressure and the residue suspended in acetone and filtered. The solvent was removed under reduced pressure and the residue was dissolved in chloroform. The organic solution was washed with dilute sodium hydrogen carbonate solution then water and was dried ($MgSO_4$). The residue was purified on silica using chloroform as eluent to give 2,3 dihydro-2-(2-pyridyl)-[1,3]-thiazino[3,2-a]benzimidazol-4-one$\frac{1}{4}$ hydrate (1.8g,) mp 165.5-167.5 °C decomp.

Analysis:

Found: C, 63.0; H, 4.2; N, 14.7;

$C_{15}H_{11}N_3OS.\frac{1}{4}H_2O$ requires:C,63.0; H, 4.1;N,14.7%).

b) The product of step a) is reduced using diborane to give the title compound.

EXAMPLE 41

Using a procedure analogous to those hereinbefore described the following compounds of formula I are prepared:

a) 6,7-dichloro-2-(2-(3,5-dimethylpyridyl)thiazolo[3,2-a]benzimidazole;
b) 6,7-dichloro-2-(2-(4-methoxypyridyl)thiazolo[3,2-a]benzimidazole;
c) 6,7-dichloro-2-(2-(3,5-dichloropyridyl)thiazolo[3,2-a]benzimidazole;
d) 6,7-dichloro-2-(2-pyridyl)thiazolo[3,2-a]benzimidazole;
e) 2-(2-(4-methoxypyridyl)-6,7-dimethoxythiazolo[3,2-a]benzimidazole;
f) 2-(2-(3,5-dichloropyridyl)-6,7-dimethoxythiazolo[3,2-a]benzimidazole;
g) 2-(2-pyridyl)-6,7-dimethoxythiazolo[3,2-a]benzimidazole;
h) 6,7-dimethyl-2-(2-(3,5-dimethylpyridyl))thiazolo[3,2-a]benzimidazole;
i) 2-(2-(3,5-dichloropyridyl))-6,7-dimethylthiazolo[3,2-a]benzimidazole
j) 2-(2-pyridyl)-6,7-dimethylthiazolo[3,2-a]benzimidazole;
k) 2-(2-(3,5-dimethylpyridyl))thiazolo[3,2-a]benzimidazole;
l) 2-(2-(4-methoxypyridyl)thiazolo[3,2-a]benzimidazole;
m) 2-(2-(3,5-dichloropyridyl)thiazolo[3,2-a]benzimidazole;
n) 2-(2-(4-methylpyridyl))thiazolo[3,2-a]benzimidazole;
o) 2-(2-(3-hydroxypyridyl))thiazolo[3,2-a]benzimidazole;
p) 2-(2-(4-methoxy-3,5-dimethyl))thiazolo[3,2-a]benzimidazole;
q) 2-(2-(3-methylpyridyl))thiazolo[3,2-a]benzimidazole;
r) 2-(2-(4-phenylpyridyl))thiazolo[3,2-a]benzimidazole;
s) 2-(2-(6-cyanopyridyl))-2,3-dihydrothiazolo[3,2-a]benzimidazole or its S-oxide;
t) 6- or 7-methoxy-2-(2-pyridyl)thiazolo[3,2-a]benzimidazole;
u) 2-(2-(6-carbamoylpyridyl))-2,3-dihydrothiazolo[3,2-a]benzimidazole or its S-oxide;
v) 6- or 7-trifluoromethyl-2-(2-pyridyl)-thiazolo[3,2-a]benzimidazole.

**Claims**

**Claims for the following Contracting States : BE, CH, DE, FR, IT, LI, LU, NL, SE**

1. A pharmaceutical composition comprising a compound of formula

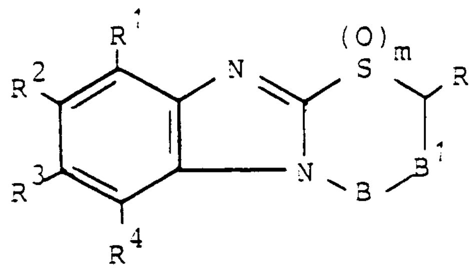

(I)

or a pharmaceutically acceptable salt thereof wherein $-B-B^1-$ represents a chain of formula

$-(CHR^5)_n-CHR^6-$ (Ia)

or

$-CR^5=CR^6-$ (Ib)

or

$-(CHR^5)_n-CR^6=$ (Ic);

R represents a mono- or bi-cyclic aryl or heteroaryl radical each optionally substituted by one or more substituents selected from the following list: lower alkyl, lower alkoxy, halogen, alkanoyloxy of 2 to 7 carbon atoms, lower alkoxycarbonyl, halolower alkyl, hydroxy, cyano, amino, mono- or diloweralkyl amino, lower alkanoylamino, carboxy, carboxyloweralkyl, hydroxylower alkyl, carbamoyl, carbamoyloxy, lower alkyl- or aryl-carbonyl, (loweralkoxy)lower alkoxy, or phenyl, or a phenyl group itself optionally substituted by substituents as hereinbefore defined excepting phenyl;
$R^1$, $R^2$, $R^3$ and $R^4$ independently represent hydrogen, or one of the substituents listed above in connection with the group R,
$R^2$ and $R^3$ together with the carbon atoms to which they are attached may also complete a fused benzo ring,
$R^5$ and $R^6$ independently represent hydrogen or lower alkyl; n and m independently represent 0 or 1, providing that when $-B-B^1-$ has formula I (a), n is 1 and m is O then R is heteroaryl, the term "heteroaryl" means a monovalent aromatic heterocyclic group in which the ring heteroatom or atoms is/are selected from oxygen, nitrogen and sulphur, the term 'lower' means a group containing 1 to 6 carbon atoms; and a pharmaceutically acceptable carrier.

2. A composition as claimed in Claim 1 wherein $-B-B^1-$ has formula Ia or Ic and n is O.

3. A composition as claimed in Claim 1 or Claim 2 wherein R represents a phenyl, naphthyl, pyridyl, thienyl, furyl, thiazolyl, quinolyl, isoquinolyl or indolyl group each optionally substituted as defined in Claim 1.

4. A composition as claimed in Claim 1 or Claim 2 wherein R represents phenyl, pyrid-2-yl or pyrid-3-yl each optionally substituted by lower alkyl, lower alkoxy, halogen, phenyl, halophenyl, lower alkylphenyl or lower alkoxyphenyl.

5. A composition as claimed in any one of Claims 1 to 4 wherein $R^2$ and $R^3$ are selected from hydrogen, lower alkyl, alkoxycarbonyl of 2 to 7 carbon atoms, halogen or lower alkoxy.

**6.** A compound of formula I as shown in Claim 1 or a pharmaceutically acceptable salt thereof wherein R, $R^1$, $R^2$, $R^3$, $R^4$, m and -B-$B^1$- are as defined in any one of Claims 1 to 5 for use as an anti-ulcer or anti-secretory agent.

**7.** A compound of formula I as shown in Claim 1 or a salt thereof wherein R, $R^1$, $R^2$, $R^3$, $R^4$,m and -B-$B^1$- are as defined in any one of Claims 1 to 5 with the provisos
(i) when -B-$B^1$- is a chain of formula Ia, n is 1 and m is O then R is heteroaryl,
or (ii) when -B-$B^1$- is a chain of formula Ic, n is O $R^6$ is hydrogen or lower alkyl and $R^1$ $R^2$ and $R^4$ are hydrogen, $R^3$ is hydrogen or hydroxy and R is phenyl, p-chlorophenyl, p-bromophenyl, p-tolyl, p-methoxyphenyl, p-phenylphenyl, 1-naphthyl or 2-thienyl, then m is 1.

**8.** A compound of formula I which is 2,3-dihydro-2-(2-pyridyl)thiazolo[3,2-a]benzimidazole-1-oxide ;
6,7-dichloro-2,3-dihydro-2-(6-methylpyrid-3-yl)thiazolo[3,2-a]benzimidazole;
2,3-dihydro-6,7-dimethyl-2-(2-pyridyl)thiazolo[3,2-a]benzimidazole-1-oxide;
6-chloro-2,3-dihydro-2-(2-pyridyl)thiazolo[3,2-a]benzamidazole-1-oxide;
7-chloro-2,3-dihydro-2-(2-pyridyl)thiazolo[3,2-a]benzamidazole-1-oxide;
2-(2-(5-ethylpyridyl))thiazolo[3,2-a]benzimidazole; 6- or 7-ethoxy-2-(2-pyridyl)thiazolo[3,2-a]-benzimidazole;
6,7-dichloro-2,3-dihydro-2-(2-pyridyl)thiazolo[3,2-a]benzimidazole;
2,3-dihydro-6-methyl-2-(2-pyridyl)thiazolo[3,2-a]benzimidazole-1-oxide;
2,3-dihydro-7-methyl-2-(2-pyridyl)thiazolo[3,2-a]benzimidazole-1-oxide;
2,3-dihydro-7-methoxycarbonyl-6-methyl-2-(2-pyridyl)thiazolo[3,2-a]benzimidazole-1-oxide;
2,3-dihydro-6-methoxycarbonyl-7-methyl-2-(2-pyridyl)thiazolo[3,2-a]benzimidazole-1-oxide;
2,3-dihydro-6-methoxycarbonyl-2-(2-pyridyl)thiazolo[3,2-a]benzimidazole-1-oxide;
2,3-dihydro-7-methoxycarbonyl-2-(2-pyridyl)thiazolo[3,2-a]benzimidazole-1-oxide,
2,3-dihydro-2-[2-(6-phenylpyridyl)]thiazolo[3,2-a]benzimidazole; or a pharmaceutically acceptable salt thereof.

**9.** A compound of formula I which is 2,3-Dihydro-2-(2-pyridyl)thiazolo[3,2-a]benzimidazole;
2,3-dihydro-2-phenylthiazolo[3,2-a]benzimidazole;
2,3-dihydro-2-phenylthiazolo[3,2-a]benzimidazole-1-oxide;
2,3-dihydro-2-(6-methylpyrid-3-yl)thiazolo[3,2-a]benzimidazole;
2,3-dihydro-6-methyl-2-(2-pyridyl)thiazolo[3,2-a]benzimidazole;
2,3-dihydro-7-methyl-2-(2-pyridyl)-thiazolo[3,2-a]benzimidazole;
2,3-dihydro-6,7-dimethyl-2-(2-pyridyl)thiazolo[3,2-a]benzimidazole;
2,3-dihydro-5-methyl-2-(2-pyridyl)thiazolo[3,2-a]benzimidazole;
2,3-Dihydro-8-methyl-2-(2-pyridyl)thiazolo[3,2-a]benzimidazole;
2,3-Dihydro-8-methyl-2-(2-pyridyl)thiazolo[3,2-a]benzimidazole-1-oxide;
2,3-dihydro-2-[2-(6-methylpyridyl)]thiazolo[3,2-a]benzamidazole-1-oxide; or a pharmaceutically acceptable salt thereof.

**10.** A process for preparing a compound of formula I as claimed in Claim 7 characterised in that
a) a compound of formula II

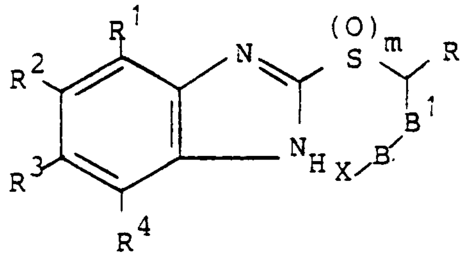

(II)

wherein -B-$B^1$-, n, m, R, $R^1$, $R^2$, $R^3$, $R^4$, $R^5$ and $R^6$ are as defined in Claim 7, and X is OH or a leaving group, providing that
(i) when -B-$B^1$- has formula Ia and n is 0 or 1, or formula Ic and n is 1 then X is not OH; and
(ii) when -B-$B^1$- has formula Ib then X is OH; is cyclised; or

b) a compound of formula (IV)

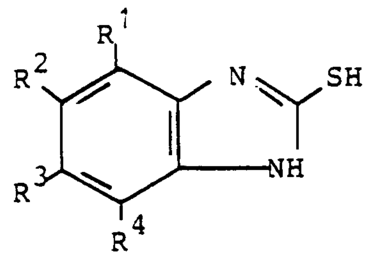

(IV)

is reacted with a compound of formula (V)

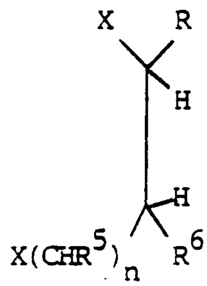

(V)

in which formulae R, $R^1$, $R^2$, $R^3$, $R^4$, $R^5$, $R^6$ and n are as defined in Claim 7 the X groups being the same or different halogens, to give a compound of formula I wherein -B-$B^1$- has formula Ia and, additionally, when X is bromine and n is O in the compound of formula V to give a compound of formula I wherein -B-$B^1$- has formula Ic wherein n is O;
or
c) a compound of formula

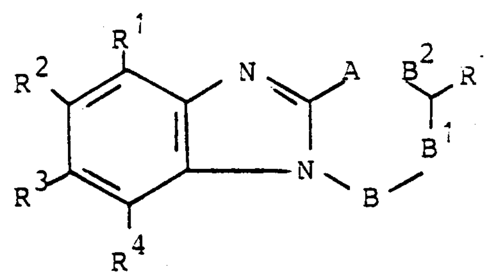

(VI)

wherein -B-$B^1$-, n, R, $R^1$, $R^2$, $R^3$, $R^4$, $R^5$ and $R^6$ are as defined in Claim 7 and one of A and $B^2$ is -SH, the other is a leaving group providing that when A is SH then $B^2$ may also represent OH,
is cyclised to give a compound of formula I wherein m is O;
or
d) reacting a compound of formula VIII

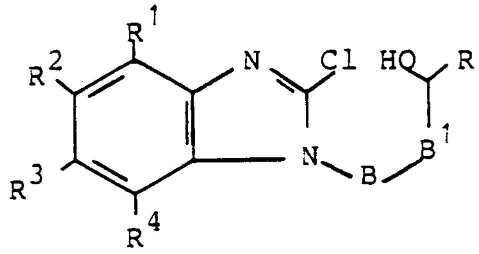

(VIII)

wherein -B-$B^1$-, $R^1$, $R^2$, $R^3$, $R^4$, $R^5$ and $R^6$ are as defined in Claim 7, with thiourea followed by

heating in the presence of an alkali metal hydroxide or $NH_4OH$ to give a compound of formula I wherein -B-$B^1$- has formula Ic;

or

e) a compound of formula

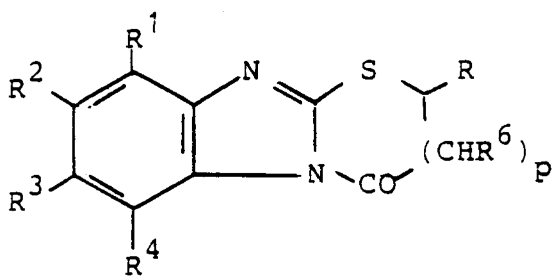

(IX)

wherein R, $R^1$, $R^2$, $R^3$, $R^4$ and $R^6$ are as defined in Claim 7 and p is 0 or 1 with the proviso that when p is 1, R is heteroaryl, is reduced to give a compound of formula I wherein m is O, -B-$B^1$ - has formula-Ia and either n is 1 and $R^5$ is hydrogen or n is O and $R^6$ is hydrogen;

or

f) a compound of formula

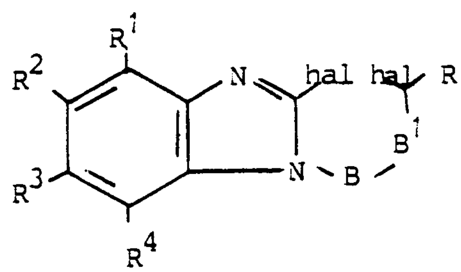

(XI)

wherein -B-$B^1$-, hal, n, R, $R^1$, $R^2$, $R^3$, $R^4$, $R^5$ and $R^6$ are as defined in Claim 7, is reacted with

(i) an alkali metal sulphide or hydrosulphide,

(ii) ammonium sulphide or polysulphide or

(iii) $H_2S$ in the presence of a tertiary amine to give a compound of formula I wherein m is O;

or

g) a compound of formula

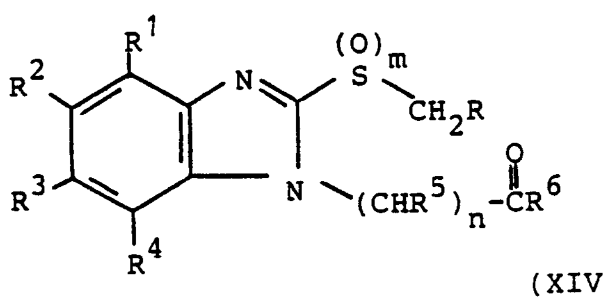

(XIV)

wherein R, $R^1$, $R^2$, $R^3$, $R^4$, $R^5$, $R^6$, m and n are as herein defined in Claim 7, is cyclised to give a compound of formula I wherein -B-$B^1$- has formula Ic;

or

h) a compound of formula I wherein m is O is oxidised to the corresponding oxide of formula I wherein m is 1 or the compound of formula I wherein m is 1 is reduced to give a compound of formula I where m is O;
or
i) a compound of formula I in free base form is converted to an acid addition or quaternary ammonium salt or an acid addition salt is neutralized to give the free base form.

**Claims for the following Contracting State : AT**

**1.** A process for preparing a compound of formula

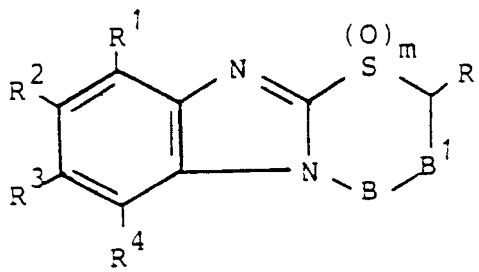

(I)

or a salt thereof, wherein $-B-B^1-$ represents a chain of formula

$-(CHR^5)_n-CHR^6-$ (Ia)

or

$-CR^5=CR^6-$ (Ib)

or

$-(CHR^5)_n-CR^6=$ (Ic);

R represents a mono- or bi-cyclic aryl or heteroaryl radical each optionally substituted by one or more substituents selected from the following list: lower alkyl, lower alkoxy, halogen, alkanoyloxy of 2 to 7 carbon atoms, lower alkoxycarbonyl, halolower alkyl, hydroxy, cyano, amino, mono- or diloweralkyl amino, lower alkanoylamino, carboxy, carboxyloweralkyl, hydroxylower alkyl, carbamoyl, carbamoyloxy, lower alkyl- or aryl-carbonyl, (loweralkoxy)lower alkoxy, or phenyl, or a phenyl group itself optionally substituted by substituents as hereinbefore defined excepting phenyl;
$R^1$, $R^2$, $R^3$ and $R^4$ independently represent hydrogen, or one of the substituents listed above in connection with the group R,
$R^2$ and $R^3$ together with the carbon atoms to which they are attached may also complete a fused benzo ring,
$R^5$ and $R^6$ independently represent hydrogen or lower alkyl; n and m independently represent 0 or 1, the term "heteroaryl" means a monovalent aromatic heterocyclic group in which the ring heteroatom or atoms is/are selected from oxygen, nitrogen and sulphur, the term 'lower' means a group containing 1 to 6 carbon atoms, with the provisos
(i) when $-B-B^1-$ is a chain or formula Ia, n is 1 and m is O then R is heteroaryl,
or (ii) when $-B-B^1-$ is a chain or formula Ic, n is O $R^6$ is hydrogen or lower alkyl and $R^1$ $R^2$ and $R^4$ are hydrogen, $R^3$ is hydrogen or hydroxy and R is phenyl, p-chlorophenyl, p-bromophenyl, p-tolyl, p-methoxyphenyl, p-phenylphenyl, 1-naphthyl or 2-thienyl, then m is 1,
characterised in that

a) a compound of formula II

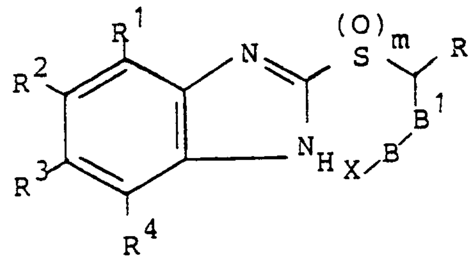

(II)

wherein -B-$B^1$-, n, m, R, $R^1$, $R^2$, $R^3$, $R^4$, $R^5$ and $R^6$ are as defined above, and X is OH or a leaving group, providing that

(i) when -B-$B^1$- has formula Ia and n is 0 or 1, or formula Ic and n is 1 then X is not OH; and

(ii) when -B-$B^1$- has formula Ib then X is OH; is cyclised; or

b) a compound of formula (IV)

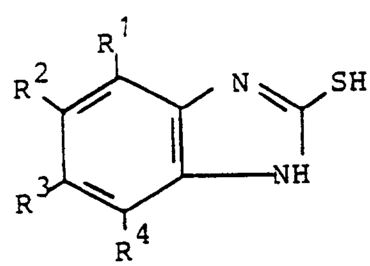

(IV)

is reacted with a compound of formula (V)

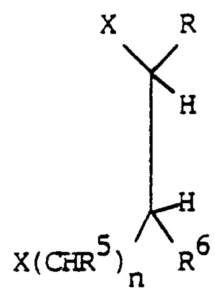

(V)

in which formulae R, $R^1$, $R^2$, $R^3$, $R^4$, $R^5$, $R^6$ and n are as defined above the X groups being the same or different halogens, to give a compound of formula I wherein -B-$B^1$- has formula Ia and, additionally, when X is bromine and n is O in the compound of formula V to give a compound of formula I wherein -B-$B^1$- has formula Ic wherein n is O.

or

c) a compound of formula

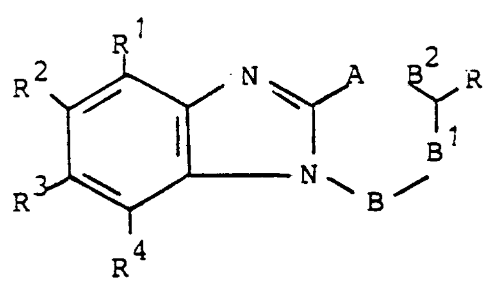

(VI)

wherein -B-B$^1$-, n, R, $R^1$, $R^2$, $R^3$, $R^4$, $R^5$ and $R^6$ are as defined above and one of A and $B^2$ is -SH, the other is a leaving group providing that when A is SH then $B^2$ may also represent OH,
is cyclised to give a compound of formula I wherein m is O;
or
d) reacting a compound of formula VIII

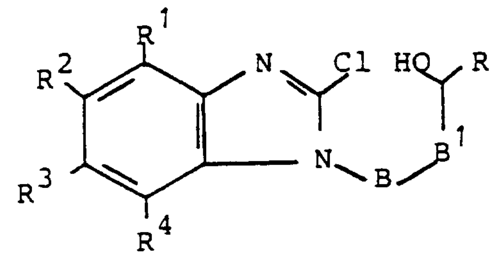

(VIII)

wherein -B-B$^1$-, $R^1$, $R^2$, $R^3$, $R^4$, $R^5$ and $R^6$ are as defined above, with thiourea followed by heating in the presence of an alkali metal hydroxide or $NH_4OH$ to give a compound of formula I wherein -B-B$^1$- has formula Ic
or
e) a compound of formula

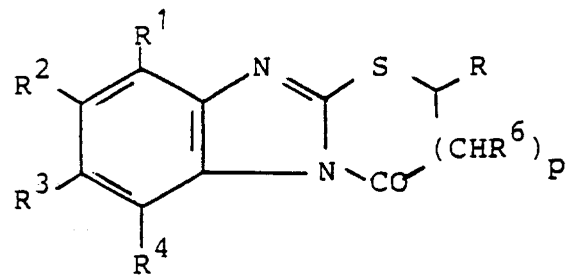

(IX)

wherein R, $R^1$, $R^2$, $R^3$, $R^4$ and $R^6$ are as defined above and p is 0 or 1 with the proviso that when p is 1, R is heteroaryl, is reduced to give a compound of formula I wherein m is O, -B-B$^1$ - has formula Ia and either n is 1 and $R^5$ is hydrogen or n is O and $R^6$ is hydrogen;
or
f) a compound of formula

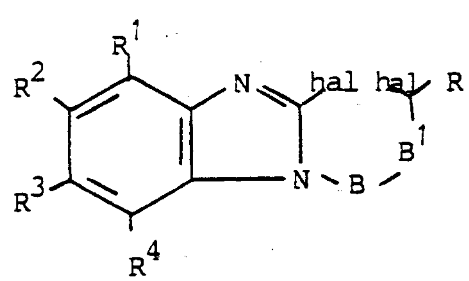

(XI)

wherein -B-B$^1$-, hal, n, R, $R^1$, $R^2$, $R^3$, $R^4$, $R^5$ and $R^6$ are as defined above, is reacted with
(i) an alkali metal sulphide or hydrosulphide,
(ii) ammonium sulphide or polysulphide or
(iii) $H_2S$ in the presence of a tertiary amine to give a compound of formula I wherein m is O;
or

g) a compound of formula

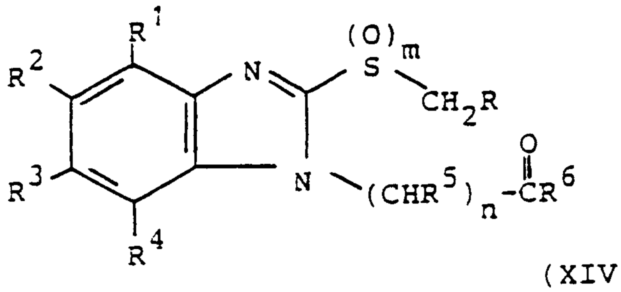

(XIV)

wherein R, $R^1$, $R^2$, $R^3$, $R^4$, $R^5$, $R^6$, m and n are as herein defined above, is cyclised to give a compound of formula I wherein -B-$B^1$- has formula Ic;

or

h) a compound of formula I wherein m is O is oxidised to the corresponding oxide of formula I wherein m is 1 or the compound of formula I wherein m is 1 is reduced to give a compound of formula I where m is O;

or

i) a compound of formula I in free base form is converted to an acid addition or quaternary ammonium salt or an acid addition salt is neutralized to give the free base form.

**2.** A process as claimed in Claim 1 wherein -B-$B^1$- has formula Ia or Ic or n is O.

**3.** A process as claimed in Claim 1 or Claim 2 wherein R represents a phenyl, naphthyl, pyridyl, thienyl, furyl, thiazolyl, quinolyl, isoquinolyl or indolyl group each optionally substituted as defined in Claim 1.

**4.** A process as claimed in Claim 1 or Claim 2 wherein R represents phenyl, pyrid-2-yl or pyrid-3-yl each optionally substituted by lower alkyl, lower alkoxy, halogen, phenyl, halophenyl, lower alkylphenyl or lower alkoxyphenyl.

**5.** A process as claimed in any one of Claims 1 to 4 wherein $R^2$ and $R^3$ are selected from hydrogen, lower alkyl, alkoxycarbonyl of 2 to 7 carbon atoms, halogen or lower alkoxy.

**6.** A process as claimed in Claim 1 in which the compound of formula I prepared is 2,3-dihydro-2-(2-pyridyl)thiazolo[3,2-a]benzimidazole-1-oxide;
6,7-dichloro-2,3-dihydro-2-(6-methylpyrid-3-yl) thiazolo[3,2-a]benzimidazole;
2,3-dihydro-6,7-dimethyl-2-(2-pyridyl)-thiazolo[3,2-a] benzimidazole-1-oxide;
6-chloro-2,3-dihydro-2-(2-pyridyl)thiazolo[3,2-a]benzamidazole-1-oxide;
7-chloro-2,3-dihydro-2-(2-pyridyl)thiazolo[3,2-a]benzamidazole-1-oxide;
2-(2-(5-ethylpyridyl))thiazolo[3,2-a]benzimidazole;
6-ethoxy-2-(2-pyridyl)thiazolo[3,2-a]benzimidazole;
7-ethoxy-2-(2-pyridyl)thiazolo[3,2-a]benzimidazole;
6,7-dichloro-2,3-dihydro-2-(2-pyridyl)thiazolo[3,2-a]benzimidazole;
2,3-dihydro-6-methyl-2-(2-pyridyl)thiazolo[3,2-a]benzimidazole-1-oxide;
2,3-dihydro-7-methyl-2-(2-pyridyl)thiazolo[3,2-a]benzimidazole-1-oxide;
2,3-dihydro-7-methoxycarbonyl-6-methyl-2-(2-pyridyl)thiazolo[3,2-a]benzimidazole-1-oxide;
2,3-dihydro-6-methoxycarbonyl-7-methyl-2-(2-pyridyl)thiazolo[3,2-a]benzimidazole-1-oxide;
2,3-dihydro-6-methoxycarbonyl-2-(2-pyridyl)thiazolo[3,2-a]benzimidazole-1-oxide;
2,3-dihydro-7-methoxycarbonyl-2-(2-pyridyl)thiazolo[3,2-a]benzimidazole-1-oxide;
2,3-dihydro-2-[2-(6-phenylpyridyl)]thiazolo[3,2-a]benzimidazole; or a pharmaceutically acceptable salt thereof.

**7.** A process as claimed in Claim 1 in which the compound of formula I prepared is 2,3-dihydro-2-(2-pyridyl)thiazolo[3,2-a]benzimidazole;
2,3-dihydro-2-phenylthiazolo[3,2-a]benzimidazole;

2,3-dihydro-2-phenylthiazolo[3,2-a]benzimidazole-1-oxide;
2,3-dihydro-2-(6-methylpyrid-3-yl)thiazolo[3,2-a]benzimidazole;
2,3-dihydro-6-methyl-2-(2-pyridyl)thiazolo[3,2-a]benzimidazole;
2,3-dihydro-7-methyl-2-(2-pyridyl)-thiazolo[3,2-a]benzimidazole;
2,3-dihydro-6,7-dimethyl-2-(2-pyridyl)thiazolo[3,2-a] benzimidazole;
2,3-dihydro-5-methyl-2-(2-pyridyl)thiazolo[3,2-a]benzimidazole or a pharmaceutically acceptable salt thereof.

**8.** A process for preparing a pharmaceutical composition characterised in that a compound of formula

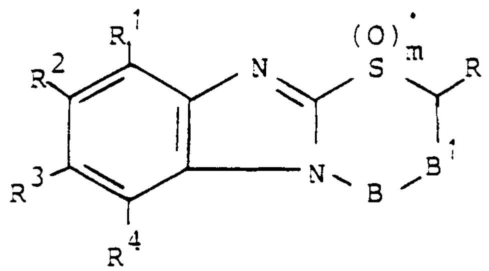

(I)

or a pharmaceutically acceptable salt thereof wherein $-B-B^1-$ represents a chain of formula

$-(CHR^5)_n-CHR^6-$ (Ia)

or

$-CR^5=CR^6-$ (Ib)

or

$-(CHR^5)_n-CR^6=$ (Ic)

R represents a mono- or bi-cyclic aryl or heteroaryl radical each optionally substituted by one or more substituents selected from the following list: lower alkyl, lower alkoxy, halogen, alkanoyloxy of 2 to 7 carbon atoms, lower alkoxycarbonyl, halo-lower alkyl, hydroxy, cyano, amino, mono- or diloweralkyl amino, lower alkanoylamino, carboxy carboxyloweralkyl, hydroxylower alkyl, carbamoyl, carbamoyloxy, lower alkyl- or aryl-carbonyl, (loweralkoxy)lower alkoxy, or phenyl, or a phenyl group itself optionally substituted by substituents as hereinbefore defined excepting phenyl; $R^1$, $R^2$, $R^3$ and $R^4$ independently represent hydrogen, or one of the substituents listed above in connection with the group R, $R^2$ and $R^3$ together with the carbon atoms to which they are attached may also complete a benzo fused ring, $R^5$ and $R^6$ independently represent hydrogen or lower alkyl, n and m independently represent 0 or 1, providing that when $-B-B^1-$ has formula I(a) n is 1 and m is O then R is heteroaryl, the term "heteroaryl" means a monovalent aromatic heterocyclic group in which the ring heteroatom or atoms is/are selected from oxygen, nitrogen and sulphur, the term 'lower' means a group containing 1 to 6 carbon atoms; is brought into a form suitable for therapeutic administration.

**9.** A process as claimed in Claim 8 wherein R represents a phenyl, naphthyl, pyridyl, thienyl, furyl, thiazolyl, quinolyl, isoquinolyl or indolyl group each optionally substituted as defined in Claim 8.

**10.** A process as claimed in Claim 8 or Claim 9 wherein R represents phenyl, pyrid-2-yl or pyrid-3-yl each optionally substituted by lower alkyl, lower alkoxy, halogen, phenyl, halophenyl, lower alkylphenyl or lower alkoxyphenyl.

**Patentansprüche**
**Patentansprüche für folgende Vertragsstaaten : BE, CH, DE, FR, IT, LI, LU, NL, SE**

1. Pharmazeutische Zusammensetzung umfassend eine Verbindung der Formel

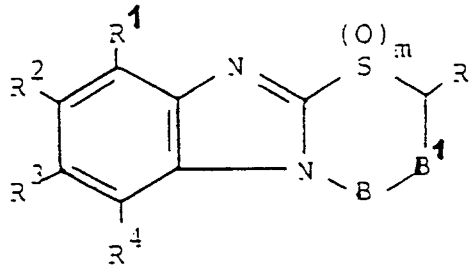

(I)

oder ein pharmazeutisch annehmbares Salz hievon, worin $-B-B^1-$ eine Kette der Formel

$-(CHR^5)_n-CHR^6-$ (Ia)

oder

$-CR^5=CR^6-$ (Ib)

oder

$-(CHR^5)_n-CR^6=$ (Ic)

darstellt,

R einen mono- oder bicyclischen Aryl- oder Heteroarylrest bedeutet, Jeweils gegebenenfalls durch einen oder mehrere Substituenten ausgewählt aus der folgenden Gruppe substituiert: nied.Alkyl, nied.Alkoxy, Halogen, Alkanoyloxy mit 2 bis 7 Kohlenstoffatomen, nied.Alkoxycarbonyl, Halogen-nied.alkyl, Hydroxy, Cyano, Amino, Mono- oder Di-nied.alkylamino, nied.Alkanoylamino, Carboxy, Carboxy-nied.alkyl, Hydroxy-nied.alkyl, Carbamoyl, Carbamoyloxy, nied.Alkyl- oder Arylcarbonyl, (nied.Alkoxy)-nied.alkoxy oder Phenyl oder eine Phenylgruppe, die selbst gegebenenfalls durch Substituenten, wie oben definiert, ausgenommen Phenyl, substituiert ist;

$R^1$, $R^2$, $R^3$ und $R^4$ unabhängig Wasserstoff oder einen der oben im Zusammenhang mit der Gruppe R angegebenen Substituenten darstellen;

$R^2$ und $R^3$ zusammen mit den Kohlenstoffatomen, an die sie gebunden sind, auch einen kondensierten Benzoring vervollständigen können;

$R^5$ und $R^6$ unabhängig Wasserstoff oder nied.Alkyl bedeuten;

n und m unabhängig Null oder 1 sind, mit der Maßgabe, daß, wenn $-B-B^1-$ die Formel (Ia) aufweist, n 1 ist und m Null ist, dann R Heteroaryl bedeutet, wobei der Ausdruck "Heteroaryl" eine einwertige aromatische heterocyclische Gruppe bedeutet, in der das oder die Ringheteroatome ausgewählt ist/sind aus Sauerstoff, Stickstoff und Schwefel, und der Ausdruck "nied." eine Gruppe mit 1 bis 6 Kohlenstoffatomen bedeutet, und einen pharmazeutisch annehmbaren Träger.

2. Zusammensetzung nach Anspruch 1, worin $-B-B^1-$ die Formel (Ia) oder (Ic) aufweist und n Null ist.

3. Zusammensetzung nach Anspruch 1 oder 2, worin R eine Phenyl-, Naphthyl-, Pyridyl-, Thienyl-, Furyl-, Thiazolyl-, Chinolyl-, Isochinolyl- oder Indolylgruppe, jeweils gegebenenfalls substituiert wie in Anspruch 1 definiert, bedeutet.

4. Zusammensetzung nach Anspruch 1 oder 2, worin R Phenyl, Pyrid-2-yl oder Pyrid-3-yl, jeweils gegebenenfalls substituiert durch nied.Alkyl, nied.Alkoxy, Halogen, Phenyl, Halogenphenyl, nied.Alkylphenyl oder nied.Alkoxyphenyl bedeutet.

5. Zusammensetzung nach einem der Ansprüche 1 bis 4, worin $R^2$ und $R^3$ ausgewählt sind aus Wasserstoff, nied.Alkyl, Alkoxycarbonyl mit 2 bis 7 Kohlenstoffatomen, Halogen oder nied.Alkoxy.

6. Verbindung der Formel (I), wie in Anspruch 1 gezeigt, oder ein pharmazeutisch annehmbares Salz hievon, worin R, $R^1$, $R^2$, $R^3$, $R^4$, m und -B-$B^1$- wie in einem der Ansprüche 1 bis 5 definiert sind, zur Verwendung als Antiulcus- oder antisekretorisches Mittel.

7. Verbindung der Formel (I), wie in Anspruch 1 gezeigt, oder ein Salz hievon, worin R, $R^1$, $R^2$, $R^3$, $R^4$, m und -B-$B^1$- wie in einem der Ansprüche 1 bis 5 definiert sind, mit den Maßgaben:
   (i) wenn -B-$B^1$- eine Kette der Formel (Ia) ist, n 1 ist und m Null ist, dann R Heteroaryl bedeutet, oder
   (ii) wenn -B-$B^1$- eine Kette der Formel (Ic) ist, n Null ist, $R^6$ Wasserstoff oder nied.Alkyl bedeutet und $R^1$, $R^2$ und $R^4$ Wasserstoff darstellen, $R^3$ Wasserstoff oder Hydroxy bedeutet und R Phenyl, p-Chlorphenyl, p-Bromphenyl, p-Tolyl, p-Methoxyphenyl, p-Phenylphenyl, 1-Naphthyl oder 2-Thienyl darstellt, dann m 1 ist.

8. Verbindung der Formel (I), die 2,3-Dihydro-2-(2-pyridyl)-thiazol[3,2-a]benzimidazol-1-oxid,
   6,7-Dichlor-2,3-dihydro-2-(6-methylpyrid-3-yl)thiazol[3,2-a]benzimidazol,
   2,3-Dihydro-6,7-dimethyl-2-(2-pyridyl)-thiazol[3,2-a]benzimidazol-1-oxid
   6-Chlor-2,3-dihydro-2-(2-pyridyl)-thiazol[3,2-a)benzimidazol-1-oxid,
   7-Chlor-2,3-dihydro-2-(2-pyridyl)-thiazol[3,2-a]benzimidazol-1-oxid
   2-[2-(5-Äthylpyridyl)]-thiazol[3,2-a]benzimidazol,
   6- oder 7-Äthoxy-2-(2-pyridyl)-thiazol[3,2-a]benzimidazol,
   6,7-Dichlor-2,3-dihydro-2-(2-pyridyl)-thiazol[3,2-a]benzimidazol,
   2,3-Dihydro-6-methyl-2-(2-pyridyl)-thiazol[3,2-a]benzimidazol-1-oxid,
   2,3-Dihydro-7-methyl-2-(2-pyridyl)-thiazol[3,2-a]benzimidazol-1-oxid,
   2,3-Dihydro-7-methoxycarbonyl-6-methyl-2-(2-pyridyl)-thiazol[3,2-a]benzimidazol-1-oxid,
   2,3-Dihydro-6-methoxycarbonyl-7-methyl-2-(2-pyridyl)-thiazol[3,2-a]benzimidazol-1-oxid,
   2,3-Dihydro-6-methoxycarbonyl-2-(2-pyridyl)-thiazol[3,2-a]benzimidazol-1-oxid,
   2,3-Dihydro-7-methoxycarbonyl-2-(2-pyridyl)-thiazol[3,2-a]benzimidazol-1-oxid,
   2,3-Dihydro-2-[2-(6-phenylpyridyl)-thiazol[3,2-a)benzimidazol oder ein pharmazeutisch annehmbares Salz hievon ist.

9. Verbindung der Formel (I), die 2,3-Dihydro-2-(2-pyridyl)-thiazol[3,2-a]benzimidazol,
   2,3-Dihydro-2-phenylthiazol[3,2-a]benzimidazol,
   2,3-Dihydro-2-phenylthiazol[3,2-a]benzimidazol-1-oxid,
   2,3-Dihydro-2-(6-methylpyrid-3-yl)-thiazol[3,2-a]benzimidazol,
   2,3-Dihydro-6-methyl-2-(2-pyridyl)-thiazol[3,2-a]benzimidazol,
   2,3-Dihydro-7-methyl-2-(2-pyridyl)-thiazol[3,2-a]benzimidazol,
   2,3-Dihydro-6,7-dimethyl-2-(2-pyridyl)-thiazol[3,2-a]benzimidazol,
   2,3-Dihydro-5-methyl-2-(2-pyridyl)-thiazol[3,2-a]benzimidazol,
   2,3-Dihydro-8-methyl-2-(2-pyridyl)-thiazol[3,2-a]benzimidazol,
   2,3-Dihydro-8-methyl-2-(2-pyridyl)-thiazol[3,2-a]benzimidazol-1-oxid,
   2,3-Dihydro-2-[2-(6-methylpyridyl)-thiazol[3,2-a]benzimidazol-1-oxid
   oder ein pharmazeutisch annehmbares Salz hievon ist.

10. Verfahren zum Herstellen einer Verbindung der Formel (I), wie in Anspruch 7 beansprucht, dadurch gekennzeichnet, daß

a) eine Verbindung der Formel

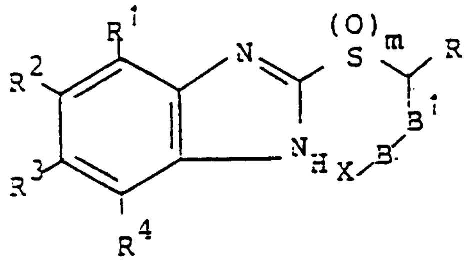

(II),

worin -B-$B^1$-, n, m, R, $R^1$, $R^2$, $R^3$, $R^4$, $R^5$ und $R^6$ wie in Anspruch 7 definiert sind und X OH oder eine Abgangsgruppe darstellt, mit der Maßgabe, daß,

(i) wenn -B-$B^1$- die Formel (Ia) hat und n Null oder 1 ist oder die Formel (Ic) hat und n 1 ist, dann X nicht OH ist, und

(ii) wenn -B-$B^1$- die Formel (Ib) hat, dann X die Bedeutung OH hat, cyclisiert wird oder

b) eine Verbindung der Formel (IV)

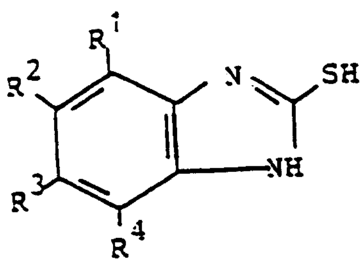

(IV)

mit einer Verbindung der Formel (V)

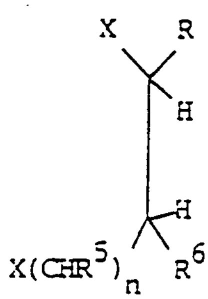

(V),

in welchen Formeln R, $R^1$, $R^2$, $R^3$, $R^4$, $R^5$, $R^6$ und n wie in Anspruch 7 definiert sind und die Gruppen X gleiche oder verschiedene Halogene sind, umgesetzt wird, wobei eine Verbindung der Formel (I), worin -B-$B^1$- die Formel (Ia) hat und, außerdem, wenn in der Verbindung der Formel (V) X Brom darstellt und n Null ist, eine Verbindung der Formel (I), worin -B-$B^1$- die Formel (Ic) hat, wobei n Null ist, erhalten wird, oder

c) eine Verbindung der Formel

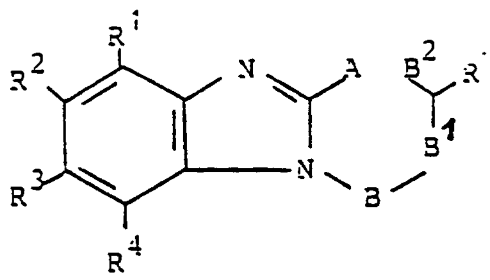

(VI),

worin -B-B$^1$-, n, R, $R^1$, $R^2$, $R^3$, $R^4$, $R^5$ und $R^6$ wie in Anspruch 7 definiert sind und eines von A und $B^2$ -SH bedeutet und das andere eine Abgangsgruppe ist, mit der Maßgabe, daß, wenn A SH ist, $B^2$ auch OH bedeuten kann, cyclisiert wird, wobei eine Verbindung der Formel (I), worin m Null ist, erhalten wird, oder

d) eine Verbindung der Formel

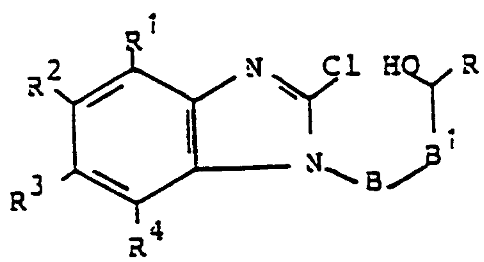

(VIII),

worin -B-B$^1$-, $R^1$, $R^2$, $R^3$, $R^4$, $R^5$ und $R^6$ wie in Anspruch 7 definiert sind, mit Thioharnstoff umgesetzt wird, gefolgt von Erhitzen in Anwesenheit eines Alkalimetallhydroxids oder von $NH_4OH$, wobei eine Verbindung der Formel (I), worin -B-B$^1$- die Formel (Ic) aufweist, erhalten wird, oder

e) eine Verbindung der Formel

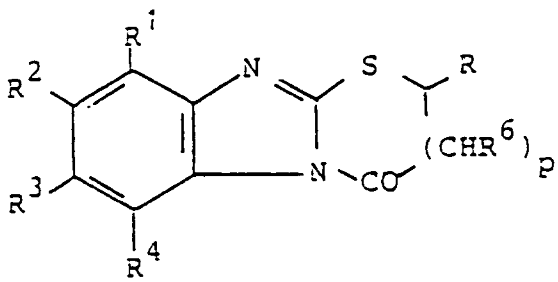

(IX),

worin R, $R^1$, $R^2$, $R^3$, $R^4$ und $R^6$ wie in Anspruch 7 definiert sind und p Null oder 1 ist, mit der Maßgabe, daß, wenn p 1 ist, R Heteroaryl darstellt, reduziert wird, wobei eine Verbindung der Formel (I), worin m Null ist, -B-B$^1$- die Formel (Ia) hat und entweder n 1 ist und $R^5$ Wasserstoff darstellt oder n Null ist und $R^6$ Wasserstoff darstellt, erhalten wird, oder

f) eine Verbindung der Formel

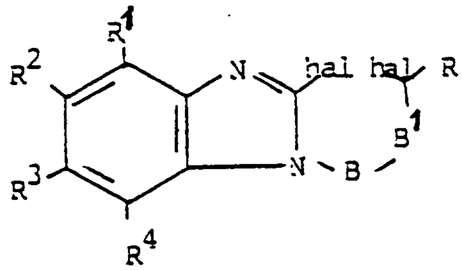

(XI),

worin -B-B$^1$-, hal, n, R, $R^1$, $R^2$, $R^3$, $R^4$ und $R^6$ wie in Anspruch 7 definiert sind, mit

(i) einem Alkalimetallsulfid oder Hydrosulfid,

(ii) Ammoniumsulfid oder Polysulfid oder

(iii) $H_2S$ in Anwesenheit eines tertiären Amins umgesetzt wird, wobei eine Verbindung der Formel (I), worin m Null ist, erhalten wird, oder

(g) eine Verbindung der Formel

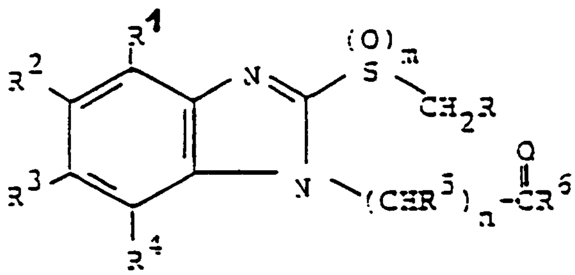

(XIV),

worin R, $R^1$, $R^2$, $R^3$, $R^4$, $R^5$, $R^6$ und n wie in Anspruch 7 definiert sind, cyclisiert wird, wobei eine Verbindung der Formel (I), worin -B-B$^1$- die Formel (Ic) aufweist, erhalten wird oder

(h) eine Verbindung der Formel (I), worin m Null ist, zum entsprechenden Oxid der Formel (I), worin m 1 ist, oxidiert wird, oder die Verbindung der Formel (I), worin m 1 ist, reduziert wird, wobei eine Verbindung der Formel (I), worin m Null ist, erhalten wird, oder (i) eine Verbindung der Formel (I) in Form der freien Base in ein Säureadditions- oder quarternäres Ammoniumsalz übergeführt oder ein Säureadditionssalz zur freien Basenform neutralisiert wird.

**Patentansprüche für folgenden Vertragsstaat : AT**

**1.** Verfahren zum Herstellen einer Verbindung der Formel

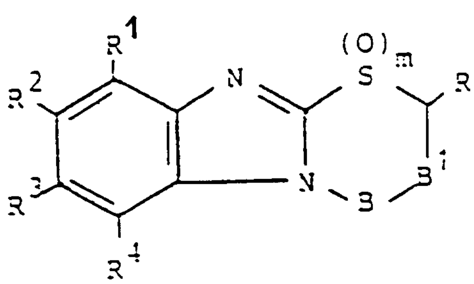

(I)

oder eines pharmazeutisch annehmbaren Salzes hievon, worin -B-B$^1$- eine Kette der Formel

$-(CHR^5)_n-CHR^6-$ (Ia)

oder

$-CR^5=CR^6-$ (Ib)

oder

$-(CHR^5)_n-CR^6=$ (Ic)

darstellt,

R einen mono- oder bicyclischen Aryl- oder Heteroarylrest bedeutet, jeweils gegebenenfalls durch einen oder mehrere Substituenten ausgewählt aus der folgenden Gruppe substituiert: nied.Alkyl, nied.Alkoxy, Halogen, Alkanoyloxy mit 2 bis 7 Kohlenstoffatomen, nied.Alkoxcarbonyl, Halogen-nied.alkyl, Hydroxy, Cyano, Amino, Mono- oder Di-nied.alkylamino, nied.Alkanoylamino, Carboxy, Carboxy-nied.alkyl, Hydroxy-nied.alkyl, Carbamoyl, Carbamoyloxy, nied.Alkyl- oder Arylcarbonyl, (nied.Alkoxy)-nied.alkoxy oder Phenyl oder eine Phenylgruppe, die selbst gegebenenfalls durch Substituenten, wie oben definiert, ausgenommen Phenyl, substituiert ist;

$R^1$, $R^2$, $R^3$ und $R^4$ unabhängig Wasserstoff oder einen der oben im Zusammenhang mit der Gruppe R angegebenen Substituenten darstellen;

$R^2$ und $R^3$ zusammen mit den Kohlenstoffatomen, an die sie gebunden sind, auch einen kondensierten Benzoring vervollständigen können;

$R^5$ und $R^6$ unabhängig Wasserstoff oder nied.Alkyl bedeuten;

n und m unabhängig Null oder 1 sind,

wobei der Ausdruck "Heteroaryl" eine einwertige aromatische heterocyclische Gruppe bedeutet, in der das oder die Ringheteroatome ausgewählt ist/sind aus Sauerstoff, Stickstoff und Schwefel, und der Ausdruck "nied." eine Gruppe mit 1 bis 6 Kohlenstoffatomen bedeutet, mit den Maßgaben, daß,

(i) wenn $-B-B^1-$ eine Kette der Formel (Ia) ist, n 1 ist und m Null ist, dann R Heteroaryl bedeutet, oder

(ii) wenn $-B-B^1-$ eine Kette der Formel (Ic) ist, n Null ist, $R^6$ Wasserstoff oder nied.Alkyl bedeutet und $R^1$, $R^2$ und $R^4$ Wasserstoff bedeuten, $R^3$ Wasserstoff oder Hydroxy ist und R Phenyl, p-Chlorphenyl, p-Bromphenyl, p-Tolyl, P-Methoxyphenyl, p-Phenylphenyl, 1-Naphthyl oder 2-Thienyl ist, dann m 1 ist,

dadurch gekennzeichnet, daß

a) eine Verbindung der Formel

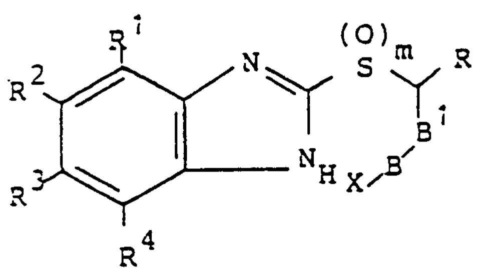

(II),

worin $-B-B^1-$, n, m, R, $R^1$, $R^2$, $R^3$, $R^4$, $R^5$ und $R^6$ wie oben definiert sind und X OH oder eine Abgangsgruppe darstellt, mit der Maßgabe, daß,

(i) wenn $-B-B^1-$ die Formel (Ia) hat und n Null oder 1 ist, oder die Formel (Ic) hat und n 1 ist, dann X nicht OH ist, und

(ii) wenn $-B-B^1-$ die Formel (Ib) hat, dann X die Bedeutung OH hat, cyclisiert wird oder

b) eine Verbindung der Formel (IV)

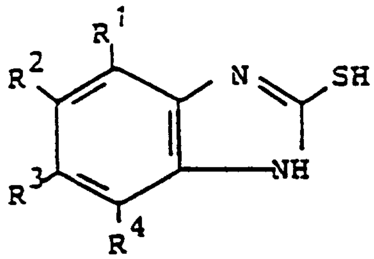

(IV)

mit einer Verbindung der Formel (V)

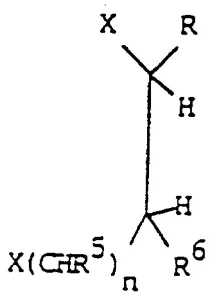

(V),

in welchen Formeln R, $R^1$, $R^2$, $R^3$, $R^4$, $R^5$, $R^6$ und n wie oben definiert sind und die Gruppen X gleiche oder verschiedene Halogene sind, umgesetzt wird, wobei eine Verbindung der Formel (I), worin $-B-B^1-$ die Formel (Ia) hat, und außerdem, wenn in der Verbindung der Formel (V) X Brom darstellt und n Null ist, eine Verbindung der Formel (I), worin $-B-B^1-$die Formel (Ic) hat, wobei n Null ist, erhalten wird, oder

c) eine Verbindung der Formel

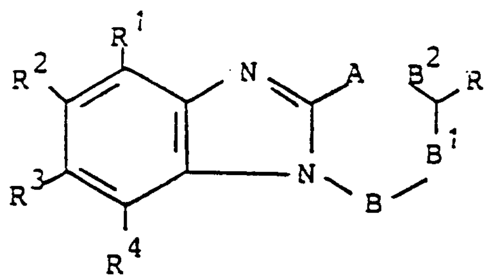

(VI),

worin $-B-B^1-$, n, R, $R^1$, $R^2$, $R^3$, $R^4$, $R^5$ und $R^6$ wie oben definiert sind und eines von A und $B^2$ -SH bedeutet und das andere eine Abgangsgruppe ist, mit der Maßgabe, daß, wenn A SH ist, $B^2$ auch OH bedeuten kann, cyclisiert wird, wobei eine Verbindung der Formel (I), worin m Null ist, erhalten wird, oder

d) eine Verbindung der Formel

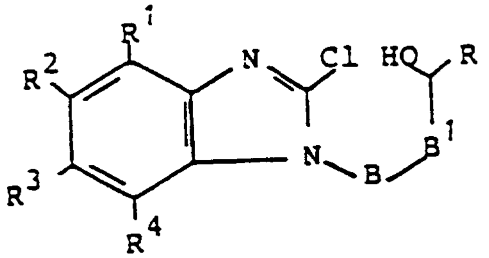

(VIII),

worin -B-$B^1$-, $R^1$, $R^2$, $R^3$, $R^4$, $R^5$ und $R^6$ wie oben definiert sind, mit Thioharnstoff umgesetzt wird, gefolgt von Erhitzen in Anwesenheit eines Alkalimetallhydroxids oder von $NH_4OH$, wobei eine Verbindung der Formel (I), worin -B-$B^1$- die Formel (Ic) aufweist, erhalten wird, oder

e) eine Verbindung der Formel

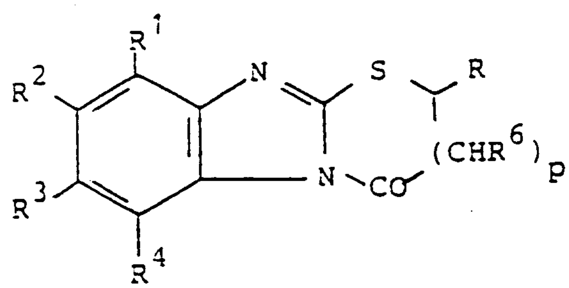

(IX),

worin R, $R^1$, $R^2$, $R^3$, $R^4$ und $R^6$ wie oben definiert sind und p Null oder 1 ist, mit der Maßgabe, daß, wenn p 1 ist, R Heteroaryl darstellt, reduziert wird, wobei eine Verbindung der Formel (I), worin m Null ist, -B-$B^1$- die Formel (Ia) hat und entweder n 1 ist und $R^5$ Wasserstoff darstellt oder n Null ist und $R^6$ Wasserstoff darstellt, erhalten wird, oder

f) eine Verbindung der Formel

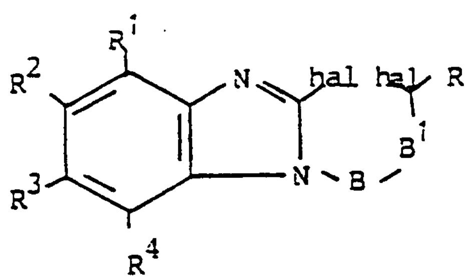

(XI),

worin -B-$B^1$-, hal, n, R, $R^1$, $R^2$, $R^3$, $R^4$ und $R^6$ wie oben definiert sind, mit

(i) einem Alkalimetallsulfid oder Hydrosulfid,

(ii) Ammoniumsulfid oder Polysulfid oder

(iii) $H_2S$ in Anwesenheit eines tertiären Amins umgesetzt wird, wobei eine Verbindung der Formel (I), worin m Null ist, erhalten wird, oder

(g) eine Verbindung der Formel

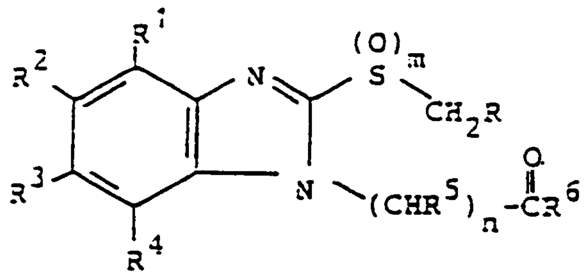

(XIV),

worin R, $R^1$, $R^2$, $R^3$, $R^4$, $R^5$, $R^6$ und n, wie oben definiert sind, cyclisiert wird, wobei eine Verbindung der Formel (I), worin -B-$B^1$- die Formel (Ic) aufweist, erhalten wird oder (h) eine Verbindung der Formel (I), worin m Null ist, zum entsprechenden Oxid der Formel (I), worin m 1 ist, oxidiert wird, oder die Verbindung der Formel (I), worin m 1 ist, reduziert wird, wobei eine Verbindung der Formel (I), worin m Null ist, erhalten wird, oder (i) eine Verbindung der Formel (I) in Form der freien Base in ein Säureadditions- oder quaternäres Ammoniumsalz übergeführt oder ein Säureadditionssalz zur freien Basenform neutralisiert wird.

**2.** Verfahren nach Anspruch 1, worin -B-$B^1$- die Formel (Ia) oder (Ic) aufweist oder n Null ist.

**3.** Verfahren nach Anspruch 1 oder 2, worin R eine Phenyl-, Naphthyl-, Pyridyl-, Thienyl-, Furyl-, Thiazolyl-, Chinolyl-, Isochinolyl- oder Indolylgruppe, jeweils gegebenenfalls substituiert wie in Anspruch 1 definiert, bedeutet.

**4.** Verfahren nach Anspruch 1 oder 2, worin R Phenyl, Pyrid-2-yl oder Pyrid-3-yl, jeweils gegebenenfalls substituiert durch nied.Alkyl, nied.Alkoxy, Halogen, Phenyl, Halogenphenyl, nied.Alkylphenyl oder nied.Alkoxyphenyl, bedeutet.

**5.** Verfahren nach einem der Ansprüche 1 bis 4, worin $R^2$ und $R^3$ ausgewählt sind aus Wasserstoff, nied.Alkyl, Alkoxycarbonyl mit 2 bis 7 Kohlenstoffatomen, Halogen oder nied.Alkoxy.

**6.** Verfahren nach Anspruch 1, worin die hergestellte Verbindung der Formel (I)
2,3-Dihydro-2-(2-pyridyl)-thiazol[3,2-a]benzimidazol-1-oxid,
6,7-Dichlor-2,3-dihydro-2-(6-methylpyrid-3-yl)thiazol[3,2-a]benzimidazol,
2,3-Dihydro-6,7-dimethyl-2-(2-pyridyl)-thiazol[3,2-a]benzimidazol-1-oxid
6-Chlor-2,3-dihydro-2-(2-pyridyl)-thiazol[3,2-a]benzimidazol-1-oxid,
7-Chlor-2,3-dihydro-2-(2-pyridyl)-thiazol[3,2-a]benzimidazol-1-oxid
2-[2-(5-Äthylpyridyl)]-thiazol[3,2-a)benzimidazol,
6-Äthoxy-2-(2-pyridyl)-thiazol[3,2-a]benzimidazol,
7-Äthoxy-2-(2-pyridyl)-thiazol[3,2-a]benzimidazol,
6,7-Dichlor-2,3-dihydro-2-(2-pyridyl)-thiazol[3,2-a]benzimidazol,
2,3-Dihydro-6-methyl-2-(2-pyridyl)-thiazol[3,2-a]benzimidazol-1-oxid,
2,3-Dihydro-7-methyl-2-(2-pyridyl)-thiazol[3,2-a]benzimidazol-1-oxid,
2,3-Dihydro-7-methoxycarbonyl-6-methyl-2-(2-pyridyl)-thiazol[3,2-a]benzimidazol-1-oxid,
2,3-Dihydro-6-methoxycarbonyl-7-methyl-2-(2-pyridyl)-thiazol[3,2-a]benzimidazol-1-oxid,
2,3-Dihydro-6-methoxycarbonyl-2-(2-pyridyl)-thiazol[3,2-a]benzimidazol-1-oxid,
2,3-Dihydro-7-methoxycarbonyl-2-(2-pyridyl)-thiazol[3,2-a]benzimidazol-1-oxid,
2,3-Dihydro-2-[2-(6-phenylpyridyl)-thiazol[3,2-a]benzimidazol oder ein pharmazeutisch annehmbares Salz hievon ist.

**7.** Verfahren nach Anspruch 1, worin die hergestellte Verbindung der Formel (I)
2,3-Dihydro-2-(2-pyridyl)-thiazol[3,2-a]benzimidazol,
2,3-Dihydro-2-phenylthiazol(3,2-a]benzimidazol,
2,3-Dihydro-2-phenylthiazol[3,2-a]benzimidazol-1-oxid,
2,3-Dihydro-2-(6-methylpyrid-3-yl)-thiazol[3,2-a]benzimidazol,

2,3-Dihydro-6-methyl-2-(2-pyridyl)-thiazol[3,2-a]benzimidazol,
2,3-Dihydro-7-methyl-2-(2-pyridyl)-thiazol[3,2-a]benzimidazol,
2,3-Dihydro-6,7-dimethyl-2-(2-pyridyl)-thiazol[3,2-a]benzimidazol,
2,3-Dihydro-5-methyl-2-(2-pyridyl)-thiazol[3,2-a]benzimidazol oder ein pharmazeutisch annehmbares Salz hievon ist.

**8.** Verfahren zum Herstellen einer pharmazeutischen Zusammensetzung, dadurch gekennzeichnet, daß eine Verbindung der Formel

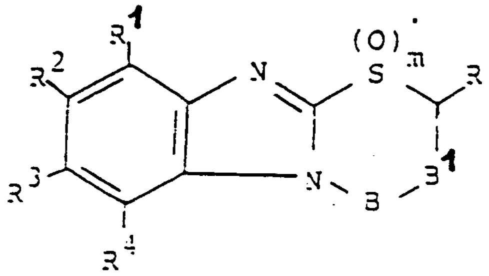

(I)

oder ein pharmazeutisch annehmbares Salz hievon, worin -B-$B^1$- eine Kette der Formel

$-(CHR^5)_n-CHR^6-$ (Ia)

oder

$-CR^5=CR^6-$ (Ib)

oder

$-(CHR^5)_n-CR^6=$ (Ic)

darstellt,

R einen mono- oder bicyclischen Aryl- oder Heteroarylrest bedeutet, jeweils gegebenenfalls durch einen oder mehrere Substituenten ausgewählt aus der folgenden Gruppe substituiert: nied.Alkyl, nied.Alkoxy, Halogen, Alkanoyloxy mit 2 bis 7 Kohlenstoffatomen, nied.Alkoxycarbonyl, Halogen-nied.alkyl, Hydroxy, Cyano, Amino, Mono- oder Di-nied.alkylamino, nied.Alkanoylamino, Carboxy, Carboxy-nied.alkyl, Hydroxy-nied.alkyl, Carbamoyl, Carbamoyloxy, nied.Alkyl- oder Arylcarbonyl, (nied.Alkoxy)-nied.alkoxy oder Phenyl oder eine Phenylgruppe, die selbst gegebenenfalls durch Substituenten, wie oben definiert, ausgenommen Phenyl, substituiert ist;

$R^1$, $R^2$, $R^3$ und $R^4$ unabhängig Wasserstoff oder einen der oben im Zusammenhang mit der Gruppe R angegebenen Substituenten darstellen;

$R^2$ und $R^3$ zusammen mit den Kohlenstoffatomen, an die sie gebunden sind, auch einen kondensierten Benzoring vervollständigen können;

$R^5$ und $R^6$ unabhängig Wasserstoff oder nied.Alkyl bedeuten;

n und m unabhängig Null oder 1 sind, mit der Maßgabe, daß, wenn -B-$B^1$- die Formel (Ia) aufweist, n 1 ist und m Null ist, dann R Heteroaryl bedeutet, wobei der Ausdruck "Heteroaryl" eine einwertige aromatische heteroccclische Gruppe bedeutet, in der das oder die Ringheteroatome ausgewählt ist/sind aus Sauerstoff, Stickstoff und Schwefel, und der Ausdruck "nied." eine Gruppe mit 1 bis 6 Kohlenstoffatomen bedeutet,

in eine für therapeutische Verabreichung geeignete Form gebracht wird.

**9.** Verfahren nach Anspruch 8, worin R eine Phenyl-, Naphthyl-, Pyridyl-, Thienyl-, Furyl-, Thiazolyl-, Chinolyl-, Isochinolyl- oder Indolylgruppe, jeweils gegebenenfalls substituiert wie in Anspruch 8 definiert, bedeutet.

**10.** Verfahren nach Anspruch 8 oder 9, worin R Phenyl, Pyrid-2-yl oder Pyrid-3-yl, jeweils gegebenenfalls substituiert durch nied.Alkyl, nied.Alkoxy, Halogen, Phenyl, Halogenphenyl, nied.Alkylphenyl oder

nied.Alkoxyphenyl, bedeutet.

**Revendications**
**Revendications pour les Etats contractants suivants : BE, CH, DE, FR, IT, LI, LU, NL, SE**

1. Composition pharmaceutique comprenant un composé de formule

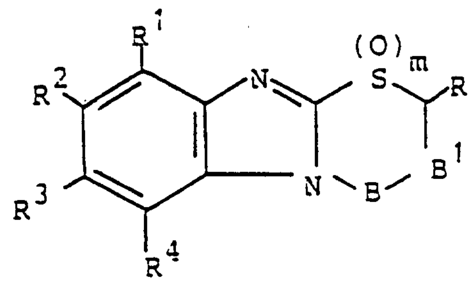

(I)

ou un sel pharmaceutiquement acceptable de celui-ci, où -B-$B^1$- représente une chaîne de formule

$-(CHR^5)_n-CHR^6-$ (Ia)

ou

$-CR^5=CR^6-$ (Ib)

ou

$-(CHR^5)_n-CR^6=$ (Ic)

R représente un radical aryle ou hétéroaryle mono- ou bicyclique dont chacun est facultativement substitué par un ou plusieurs substituants choisis dans la liste suivante : alcoyle inférieur, alcoxy inférieur, halogène, alcanoyloxy de 2 à 7 atomes de carbone, alcoxycarbonyle inférieur, haloalcoyle inférieur, hydroxyle, cyano, amino, mono- ou dialcoyl(inférieur)amino, alcanoylamino inférieur, carboxyle, carboxyalcoyle inférieur, hydroxyalcoyle inférieur, carbamoyle, carbamoyloxy, alcoyl(inférieur)- ou aryl-carbonyle, alcoxy(inférieur)alcoxy inférieur ou phényle, ou un radical phényle lui-même facultativement substitué par les substituants tels que définis ci-dessus à l'exception de phényle;

$R^1$, $R^2$, $R^3$ et $R^4$ représentent indépendamment hydrogène ou l'un des substituants énumérés ci-dessus à propos du radical R;

$R^2$ et $R^3$, conjointement avec les atomes de carbone auxquels ils sont unis, peuvent également compléter un cycle benzo condensé;

$R^5$ et $R^6$ représentent indépendamment hydrogène ou alcoyle inférieur;

n et m représentent indépendamment 0 ou 1,

avec la restriction que lorsque -B-$B^1$- est de formule I (a), n est 1 et m est 0, alors R est hétéroaryle, que le terme "hétéroaryle" désigne un radical hétérocyclique aromatique monovalent dans lequel le ou les hétéroatomes de cycle est ou sont choisis parmi oxygène, azote et soufre et que le terme "inférieur" qualifie un radical comptant 1 à 6 atomes de carbone;

et un excipient pharmaceutiquement acceptable.

2. Composition suivant la revendication 1, dans laquelle -B-$B^1$- est de formule Ia ou Ic et n est 0.

3. Composition suivant la revendication 1 ou 2, dans laquelle R représente un radical phényle, naphtyle, pyridyle, thiényle, furyle, thiazolyle, quinoléyle, isoquinoléyle ou indolyle dont chacun est facultativement substitué comme défini dans la revendication 1.

4. Composition suivant la revendication 1 ou 2, dans laquelle R représente phényle, pyrid-2-yle ou pyrid-3-yle, dont chacun est facultativement substitué par alcoyle inférieur, alcoxy inférieur, halogène, phényle, halophényle, alcoyl(inférieur)phényle ou alcoxy(inférieur)phényle.

**5.** Composition suivant l'une quelconque des revendications 1 à 4, dans laquelle $R^2$ et $R^3$ sont choisis parmi hydrogène, alcoyle inférieur, alcoxycarbonyle de 2 à 7 atomes de carbone, halogène et alcoxy inférieur.

**6.** Composé de formule I suivant la revendication 1 ou un sel pharmaceutiquement acceptable de celui-ci, dans lequel R, $R^1$, $R^2$, $R^3$, $R^4$, m et -B-$B^1$- sont tels que définis dans l'une quelconque des revendications 1 à 5, à utiliser comme agent anti-ulcéreux ou antisécrétoire.

**7.** Composé de formule I suivant la revendication 1 ou un sel de celui-ci, dans lequel R, $R^1$, $R^2$, $R^3$, $R^4$, m et -B-$B^1$- sont tels que définis dans l'une quelconque des revendications 1 à 5,
avec les restrictions que
(i) lorsque -B-$B^1$- est une chaîne de formule Ia, n est 1 et m est 0, alors R est hétéroaryle,
ou
(ii) lorsque -B-$B^1$- est une chaîne de formule Ic, n est 0, $R^6$ est hydrogène ou alcoyle inférieur, $R^1$, $R^2$ et $R^4$ sont hydrogène, $R^3$ est hydrogène ou hydroxyle et R est phényle, p-chlorophényle, p-bromophényle, p-tolyle, p-méthoxyphényle, p-phénylphényle, 1-naphtyle ou 2-thiényle, alors m est 1.

**8.** Composé de formule I qui est
le 2,3-dihydro-2-(2-pyridyl)thiazolo[3,2-a]benzimidazole-1-oxyde;
le 6,7-dichloro-2,3-dihydro-2-(6-méthylpyrid-3-yl)thiazolo[3,2-a]benzimidazole;
le 2,3-dihydro-6,7-diméthyl-2-(2-pyridyl)thiazolo[3,2-a]benzimidazole-1-oxyde;
le 6-chloro-2,3-dihydro-2-(2-pyridyl)thiazolo[3,2-a]benzimidazole-1-oxyde;
le 7-chloro-2,3-dihydro-2-(2-pyridyl)thiazolo[3,2-a]benzimidazole-1-oxyde;
le 2-[2-(5-éthylpyridyl)]thiazolo[3,2-a]benzimidazole;
le 7-éthoxy-2-(2-pyridyl)thiazolo[3,2-a]benzimidazole;
le 6,7-dichloro-2,3-dihydro-2-(2-pyridyl)thiazolo[3,2-a]benzimidazole;
le 2,3-dihydro-6-méthyl-2-(2-pyridyl)thiazolo[3,2-a]benzimidazole-1-oxyde;
le 2,3-dihydro-7-méthyl-2-(2-pyridyl)thiazolo[3,2-a]benzimidazole-1-oxyde;
le 2,3-dihydro-7-méthoxycarbonyl-6-méthyl-2-(2-pyridyl)thiazolo[3,2-a]benzimidazole-1-oxyde;
le 2,3-dihydro-6-méthoxycarbonyl-7-méthyl-2-(2-pyridyl)thiazolo[3,2-a]benzimidazole-1-oxyde;
le 2,3-dihydro-6-méthoxycarbonyl-2-(2-pyridyl)thiazolo[3,2-a]benzimidazole-1-oxyde;
le 2,3-dihydro-7-méthoxycarbonyl-2-(2-pyridyl)thiazolo[3,2-a]benzimidazole-1-oxyde;
le 2,3-dihydro-2-[2-(6-phénylpyridyl)]thiazolo[3,2-a]benzimidazole; ou
un sel pharmaceutiquement acceptable de l'un de ceux-ci.

**9.** Composé de formule I, qui est
le 2,3-dihydro-2-(2-pyridyl)thiazolo[3,2-a]benzimidazole;
le 2,3-dihydro-2-phénylthiazolo[3,2-a]benzimidazole;
le 2,3-dihydro-2-phénylthiazolo[3,2-a]benzimidazole-1-oxyde;
le 2,3-dihydro-2-(6-méthylpyrid-3-yl)thiazolo[3,2-a]benzimidazole;
le 2,3-dihydro-6-méthyl-2-(2-pyridyl)thiazolo[3,2-a]benzimidazole;
le 2,3-dihydro-7-méthyl-2-(2-pyridyl)thiazolo[3,2-a]benzimidazole;
le 2,3-dihydro-6,7-diméthyl-2-(2-pyridyl)thiazolo[3,2-a]benzimidazole;
le 2,3-dihydro-5-méthyl-2-(2-pyridyl)thiazolo[3,2-a]benzimidazole;
le 2,3-dihydro-8-méthyl-2-(2-pyridyl)thiazolo[3,2-a]benzimidazole;
le 2,3-dihydro-8-méthyl-2-(2-pyridyl)thiazolo[3,2-a]benzimidazole-1-oxyde;
le 2,3-dihydro-2-(2-(6-méthylpyridyl)]thiazolo[3,2-a]benzimidazole-1-oxyde ou
un sel pharmaceutiquement acceptable de l'un de ceux-ci.

**10.** Procédé de préparation d'un composé de formule I suivant la revendication 7, caractérisé en ce que

a) un composé de formule II

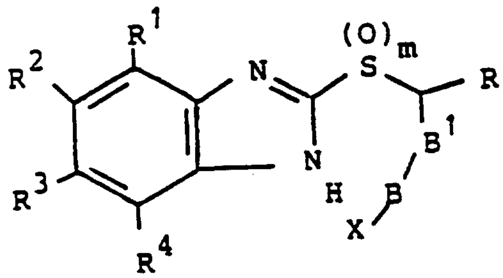

(II)

où -B-B$^1$-, n, m, R, $R^1$, $R^2$, $R^3$, $R^4$, $R^5$ et $R^6$ sont tels que définis dans la revendication 7 et X est OH ou un radical partant,
avec les restrictions que

(i) lorsque -B-B$^1$- est de formule Ia et n est 0 ou 1 ou de formule Ic et n est 1, alors X n'est pas OH; et

(ii) lorsque -B-B$^1$- est de formule Ib, alors X est OH, est cyclisé;

ou

b) un composé de formule (IV)

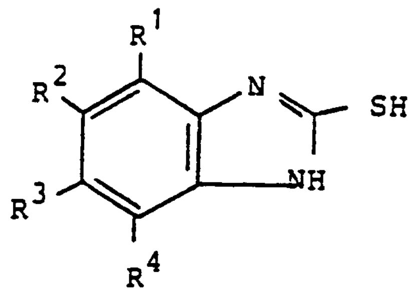

(IV)

est mis à réagir avec un composé de formule (V)

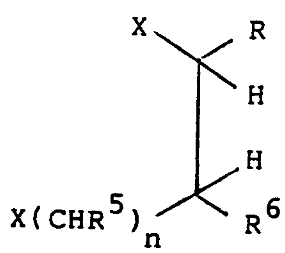

(V)

formules dans lesquelles R, $R^1$, $R^2$, $R^3$, $R^4$, $R^5$, $R^6$ et n sont tels que définis dans la revendication 7, les radicaux X étant des halogènes identiques ou différents,
pour former un composé de formule I où -B-B$^1$- est de formule Ia et de surcroît, lorsque X est brome et n est 0 dans le composé de formule V, pour former un composé de formule I où -B-B$^1$- est de formule Ic où n est 0;
ou

c) un composé de formule

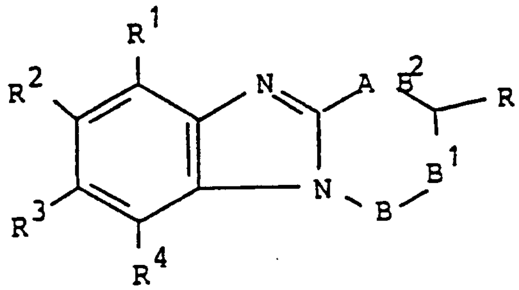

(VI)

où -B-B[1]-, n, R, $R^1$, $R^2$, $R^3$, $R^4$, $R^5$ et $R^6$ sont tels que définis dans la revendication 7 et l'un d'entre A et $B^2$ est -SH, l'autre est un radical partant
avec la restriction que lorsque A est SH, $B^2$ peut alors aussi représenter OH,
est cyclisé pour former un composé de formule I où m est 0;
ou
d) un composé de formule VIII

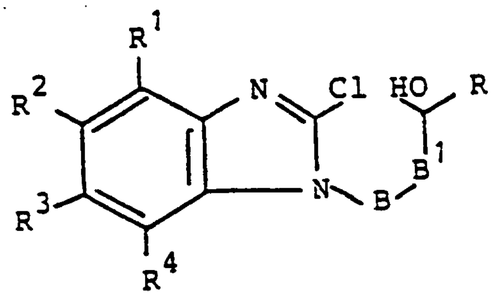

(VIII)

où -B-$B^1$-, $R^1$, $R^2$, $R^3$, $R^4$, $R^5$ et $R^6$ sont tels que définis dans la revendication 7,
est mis à réagir avec la thiourée avant un chauffage en présence d'un hydroxyde de métal alcalin ou de $NH_4OH$ pour former un composé de formule I où -B-$B^1$- est de formule Ic;
ou
e) un composé de formule

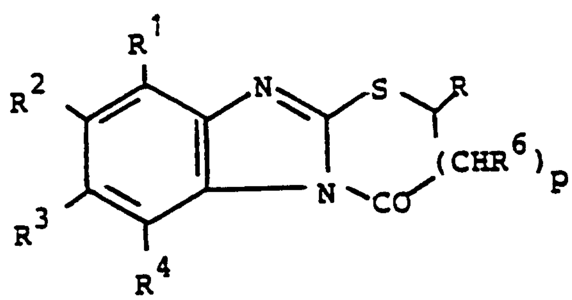

(IX)

où R, $R^1$, $R^2$, $R^3$, $R^4$ et $R^6$ sont tels que définis dans la revendication 7 et p est 0 ou 1
avec la restriction que lorsque p est 1, R est hétéroaryle, est réduit pour former un composé de formule I où m est 0, -B-$B^1$- est de formule Ia et soit n est 1 et $R^5$ est hydrogène, soit n est 0 et $R^6$ est hydrogène;
ou

f) un composé de formule

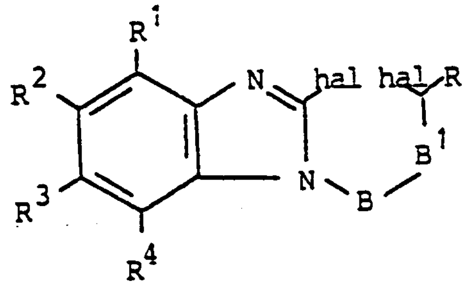

(XI)

où $-B-B^1-$, hal, n, R, $R^1$, $R^2$, $R^3$, $R^4$, $R^5$ et $R^6$ sont tels que définis dans la revendication 7, est mis à réagir avec

(i) un sulfure ou hydrosulfure de métal alcalin,

(ii) le sulfure ou polysulfure d'ammonium ou

(iii) $H_2S$ en présence d'une amine tertiaire pour former un composé de formule I où m est 0;

ou

(g) un composé de formule

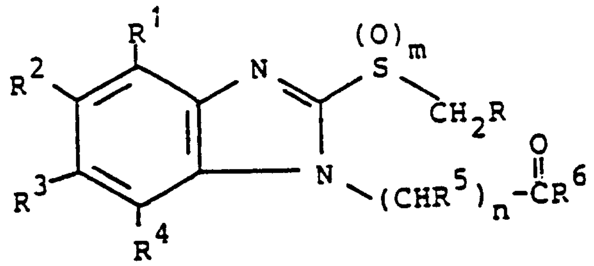

(XIV)

où R, $R^1$, $R^2$, $R^3$, $R^4$, $R^5$, $R^6$, m et n sont tels que définis dans la revendication 7, est cyclisé pour former un composé de formule I où $-B-B^1-$ est de formule Ic;

ou

(h) un composé de formule I où m est 0 est oxydé en l'oxyde correspondant de formule I où m est 1 ou le composé de formule I où m est 1 est réduit pour donner le composé de formule I où m est 0;

ou

(i) un composé de formule I sous forme de base libre est converti en un sel d'addition d'acide ou sel d'ammonium quaternaire, ou un sel d'addition d'acide est neutralisé pour donner la forme base libre.

**Revendications pour l'Etat contractant suivant : AT**

**1.** Procédé de préparation d'un composé de formule

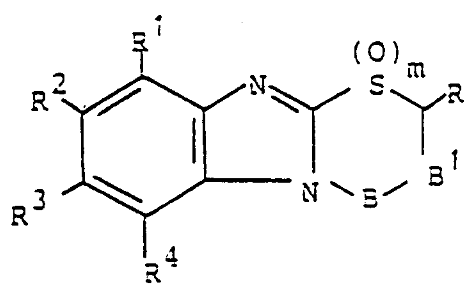

(I)

ou d'un sel de celui-ci,

où $-B-B^1-$ représente une chaîne de formule

$-(CHR^5)_n-CHR^6-$ (Ia)

ou

$-CR^5=CR^6-$ (Ib)

ou

$-(CHR^5)n-CR^6=$ (Ic)

R représente un radical aryle ou hétéroaryle mono- ou bicyclique dont chacun est facultativement substitué par un ou plusieurs substituants choisis dans la liste suivante : alcoyle inférieur, alcoxy inférieur, halogène, alcanoyloxy de 2 à 7 atomes de carbone, alcoxycarbonyle inférieur, haloalcoyle inférieur, hydroxyle, cyano, amino, mono- ou dialcoyl(inférieur)amino, alcanoylamino inférieur, carboxyle, carboxyalcoyle inférieur, hydroxyalcoyle inférieur, carbamoyle, carbamoyloxy, alcoyl(inférieur)- ou aryl-carbonyle, alcoxy(inférieur)alcoxy inférieur ou phényle, ou un radical phényle lui-même facultativement substitué par les substituants tels que définis ci-dessus à l'exception de phényle;

$R^1$, $R^2$, $R^3$ et $R^4$ représentent indépendamment hydrogène ou l'un des substituants énumérés ci-dessus à propos du radical R;

$R^2$ et $R^3$, conjointement avec les atomes de carbone auxquels ils sont unis, peuvent également compléter un cycle benzo condensé;

$R^5$ et $R^6$ représentent indépendamment hydrogène ou alcoyle inférieur;

n et m représentent indépendamment 0 ou 1,

le terme "hétéroaryle" désigne un radical hétérocyclique aromatique monovalent dans lequel le ou les hétéroatomes de cycle est ou sont sélectionnés parmi oxygène, azote et soufre et le terme "inférieur" qualifie un radical comptant 1 à 6 atomes de carbone,

avec les restrictions que

(i) lorsque $-B-B^1-$ est une chaîne de formule Ia, n est 1 et m est 0, alors R est hétéroaryle,

ou

(ii) lorsque $-B-B^1-$ est une chaîne de formule Ic, n est 0, $R^6$ est hydrogène ou alcoyle inférieur, $R^1$, $R^2$ et $R^4$ sont hydrogène, $R^3$ est hydrogène ou hydroxyle et R est phényle, p-chlorophényle, p-bromophényle, p-tolyle, p-méthoxyphényle, p-phénylphényle, 1-naphtyle ou 2-thiényle, alors m est 1,

caractérisé en ce que

a) un composé de formule II

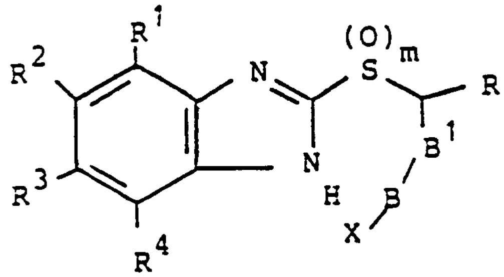

(II)

où $-B-B^1-$, n, m, R, $R^1$, $R^2$, $R^3$, $R^4$, $R^5$ et $R^6$ sont tels que définis ci-dessus et X est OH ou un radical partant,

avec les restrictions que

(i) lorsque $-B-B^1-$ est de formule Ia et n est 0 ou 1 ou de formule Ic et n est 1, alors X n'est pas OH; et

(ii) lorsque $-B-B^1-$ est de formule Ib, alors X est OH, est cyclisé;

ou

b) un composé de formule (IV)

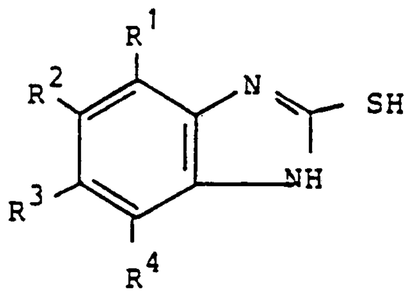

(IV)

est mis à réagir avec un composé de formule (V)

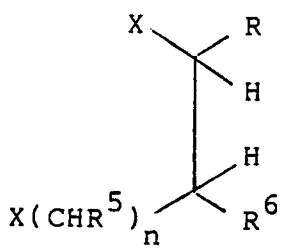

(V)

formules dans lesquelles R, $R^1$, $R^2$, $R^3$, $R^4$, $R^5$, $R^6$ et n sont tels que définis ci-dessus, les radicaux X étant des halogènes identiques ou différents,
pour former un composé de formule I où -B-$B^1$- est de formule Ia et de surcroît, lorsque X est brome et n est 0 dans le composé de formule V, pour former un composé de formule I où -B-$B^1$- est de formule Ic où n est 0;
ou
c) un composé de formule

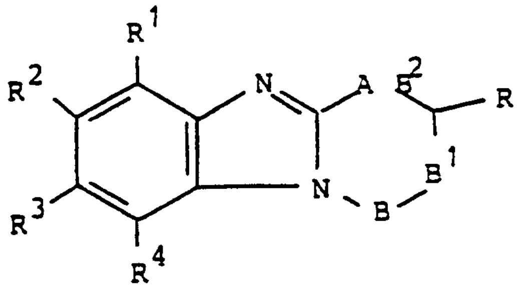

(VI)

où -B-$B^1$-, n, R, $R^1$, $R^2$, $R^3$, $R^4$, $R^5$ et $R^6$ sont tels que définis ci-dessus et l'un d'entre A et $B^2$ est -SH, l'autre est un radical partant
avec la restriction que lorsque A est SH, $B^2$ peut alors aussi représenter OH,
est cyclisé pour former un composé de formule I où m est 0;
ou

d) un composé de formule VIII

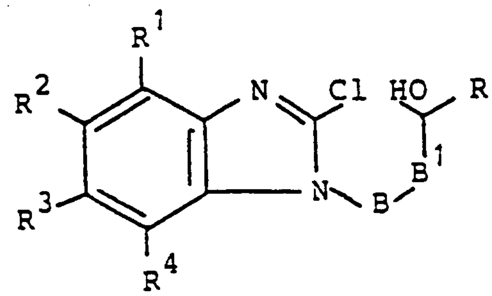

(VIII)

où -B-$B^1$-, $R^1$, $R^2$, $R^3$, $R^4$, $R^5$ et $R^6$ sont tels que définis ci-dessus, est mis a réagir avec la thiourée avant un chauffage en présence d'un hydroxyde de métal alcalin ou de $NH_4OH$ pour former un composé de formule I où -B-$B^1$- est de formule Ic;

ou

e) un composé de formule

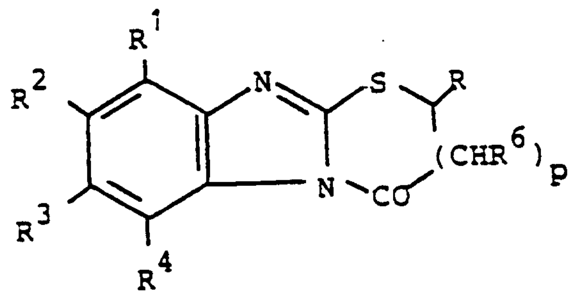

(IX)

où R, $R^1$, $R^2$, $R^3$, $R^4$ et $R^6$ sont tels que définis ci-dessus et p est 0 ou 1 avec la restriction que lorsque p est 1, R est hétéroaryle, est réduit pour former un composé de formule I où m est 0, -B-$B^1$- est de formule Ia et soit n est 1 et $R^5$ est hydrogène, soit n est 0 et $R^6$ est hydrogène;

ou

f) un composé de formule

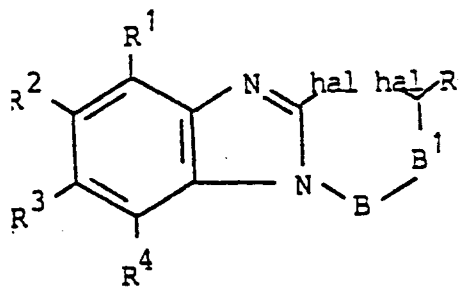

(XI)

où -B-$B^1$-, hal, n, R, $R^1$, $R^2$, $R^3$, $R^4$, $R^5$ et $R^6$ sont tels que définis ci-dessus, est mis à réagir avec

(i) un sulfure ou hydrosulfure de métal alcalin,

(ii) le sulfure ou polysulfure d'ammonium ou

(iii) $H_2S$ en présence d'une amine tertiaire pour former un composé de formule I où m est 0;

ou

(g) un composé de formule

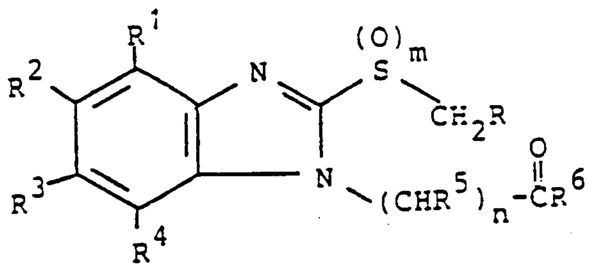

(XIV)

où R, $R^1$, $R^2$, $R^3$, $R^4$, $R^5$, $R^6$, m et n sont tels que définis ci-dessus, est cyclisé pour former un composé de formule I où -B-$B^1$- est de formule Ic;
ou
(h) un composé de formule I où m est 0 est oxydé en l'oxyde correspondant de formule I où m est 1 ou le composé de formule I où m est 1 est réduit pour donner le composé de formule I où m est 0;
ou
(i) un composé de formule I sous forme de base libre est converti en un sel d'addition d'acide ou sel d'ammonium quaternaire, ou un sel d'addition d'acide est neutralisé pour donner la forme base libre.

**2.** Procédé suivant la revendication 1, dans lequel -B-$B^1$- est de formule Ia ou Ic ou n est 0.

**3.** Procédé suivant la revendication 1 ou 2, dans lequel R représente un radical phényle, naphtyle, pyridyle, thiényle, furyle, thiazolyle, quinoléyle, isoquinoléyle ou indolyle dont chacun est facultativement substitué comme défini dans la revendication 1.

**4.** Procédé suivant la revendication 1 ou 2, dans lequel R représente phényle, pyrid-2-yle ou pyrid-3-yle, dont chacun est facultativement substitué par alcoyle inférieur, alcoxy inférieur, halogène, phényle, halophényle, alcoyl(inférieur)phényle ou alcoxy(inférieur)phényle.

**5.** Procédé suivant l'une quelconque des revendications 1 à 4, dans lequel $R^2$ et $R^3$ sont choisis parmi hydrogène, alcoyle inférieur, alcoxycarbonyle de 2 à 7 atomes de carbone, halogène et alcoxy inférieur.

**6.** Procédé suivant la revendication 1, dans lequel le composé de formule I qui est préparé est
le 2,3-dihydro-2-(2-pyridyl)thiazolo[3,2-a]benzimidazole-1-oxyde;
le 6,7-dichloro-2,3-dihydro-2-(6-méthylpyrid-3-yl)thiazolo[3,2-a]benzimidazole;
le 2,3-dihydro-6,7-diméthyl-2-(2-pyridyl)thiazolo[3,2-a]benzimidazole-1-oxyde;
le 6-chloro-2,3-dihydro-2-(2-pyridyl)thiazolo[3,2-a]benzimidazole-1-oxyde;
le 7-chloro-2,3-dihydro-2-(2-pyridyl)thiazolo[3,2-a]benzimidazole-1-oxyde;
le 2-[2-(5-éthylpyridyl)]thiazolo[3,2-a]benzimidazole;
le 6-éthoxy-2-(2-pyridyl)thiazolo[3,2-a]benzimidazole;
le 7-éthoxy-2-(2-pyridyl)thiazolo(3,2-a]benzimidazole;
le 6,7-dichloro-2,3-dihydro-2-(2-pyridyl)thiazolo[3,2-a]benzimidazole;
le 2,3-dihydro-6-méthyl-2-(2-pyridyl)thiazolo[3,2-a]benzimidazole-1-oxyde;
le 2,3-dihydro-7-méthyl-2-(2-pyridyl)thiazolo[3,2-a]benzimidazole-1-oxyde;
le 2,3-dihydro-7-méthoxycarbonyl-6-méthyl-2-(2-pyridyl)thiazolo[3,2-a]benzimidazole-1-oxyde;
le 2,3-dihydro-6-méthoxycarbonyl-7-méthyl-2-(2-pyridyl)thiazolo[3,2-a]benzimidazole-1-oxyde;
le 2,3-dihydro-6-méthoxycarbonyl-2-(2-pyridyl)thiazolo[3,2-a)benzimidazole-1-oxyde;
le 2,3-dihydro-7-méthoxycarbonyl-2-(2-pyridyl)thiazolo[3,2-a]benzimidazole-1-oxyde;
le 2,3-dihydro-2-[2-(6-phénylpyridyl)]thiazolo[3,2-a]benzimidazole; ou
un sel pharmaceutiquement acceptable de l'un de ceux-ci.

**7.** Procédé suivant la revendication 1, dans lequel le composé de formule I qui est préparé est
le 2,3-dihydro-2-(2-pyridyl)thiazolo[3,2-a]benzimidazole;
le 2,3-dihydro-2-phénylthiazolo[3,2-a]benzimidazole;

le 2,3-dihydro-2-phénylthiazolo[3,2-a]benzimidazole-1-oxyde;
le 2,3-dihydro-2-(6-méthylpyrid-3-yl)thiazolo[3,2-a]benzimidazole;
le 2,3-dihydro-6-méthyl-2-(2-pyridyl)thiazolo[3,2a]benzimidazole;
le 2,3-dihydro-7-méthyl-2-(2-pyridyl)thiazolo[3,2-a]benzimidazole;
le 2,3-dihydro-6,7-diméthyl-2-(2-pyridyl)thiazolo[3,2-a]benzimidazole;
le 2,3-dihydro-5-méthyl-2-(2-pyridyl)thiazolo[3,2-a]benzimidazole ou
un sel pharmaceutiquement acceptable de l'un de ceux-ci.

**8.** Procédé de préparation d'une composition pharmaceutique, caractérisé en ce qu'un composé de formule

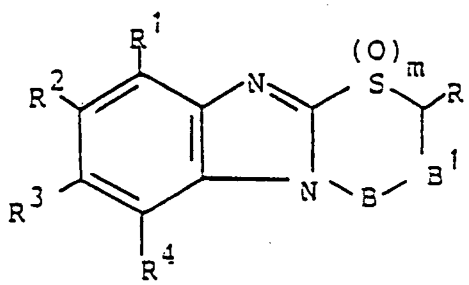

(I)

ou un sel pharmaceutiquement acceptable de celui-ci, où $-B-B^1-$ représente une chaîne de formule

$-(CHR^5)_n-CHR^6-$ (Ia)

ou

$-CR^5=CR^6-$ (Ib)

ou

$-(CHR^5)_n-CR^6=$ (Ic)

R représente un radical aryle ou hétéroaryle mono- ou bicyclique dont chacun est facultativement substitué par un ou plusieurs substituants choisis dans la liste suivante : alcoyle inférieur, alcoxy inférieur, halogène, alcanoyloxy de 2 à 7 atomes de carbone, alcoxycarbonyle inférieur, haloalcoyle inférieur, hydroxyle, cyano, amino, mono- ou dialcoyl(inférieur)amino, alcanoylamino inférieur, carboxyle, carboxyalcoyle inférieur, hydroxyalcoyle inférieur, carbamoyle, carbamoyloxy, alcoyl(inférieur)- ou aryl-carbonyle, alcoxy(inférieur)alcoxy inférieur ou phényle, ou un radical phényle lui-même facultativement substitué par les substituants tels que définis ci-dessus à l'exception de phényle;

$R^1$, $R^2$, $R^3$ et $R^4$ représentent indépendamment hydrogène ou l'un des substituants énumérés ci-dessus à propos du radical R;

$R^2$ et $R^3$, conjointement avec les atomes de carbone auxquels ils sont unis, peuvent également compléter un cycle benzo condensé;

$R^5$ et $R^6$ représentent indépendamment hydrogène ou alcoyle inférieur;

n et m représentent indépendamment 0 ou 1,

avec la restriction que lorsque $-B-B^1-$ est de formule I (a), n est 1 et m est 0, alors R est hétéroaryle, que le terme "hétéroaryle" désigne un radical hétérocyclique aromatique monovalent dans lequel le ou les hétéroatomes de cycle est ou sont choisis parmi oxygène, azote et soufre et que le terme "inférieur" qualifie un radical comptant 1 à 6 atomes de carbone;
est présenté sous une forme propre à l'administration thérapeutique.

**9.** Procédé suivant la revendication 8, dans lequel R représente un radical phényle, naphtyle, pyridyle, thiényle, furyle, thiazolyle, quinoléyle, isoquinoléyle ou indolyle dont chacun est facultativement substitué comme défini dans la revendication 8.

**10.** Procédé suivant la revendication 8 ou 9, dans lequel R représente phényle, pyrid-2-yle ou pyrid-3-yle, dont chacun est facultativement substitué par alcoyle inférieur, alcoxy inférieur, halogène, phényle, halophényle, alcoyl(inférieur)phényle ou alcoxy(inférieur)phényle.